(19) Europäisches Patentamt / European Patent Office / Office européen des brevets

(11) **EP 4 273 245 A1**

(12) **EUROPEAN PATENT APPLICATION**
published in accordance with Art. 153(4) EPC


(43) Date of publication:
**08.11.2023 Bulletin 2023/45**

(21) Application number: **21914267.6**

(22) Date of filing: **27.12.2021**

(51) International Patent Classification (IPC):
***C12N 15/113*** *(2010.01)* ***A61K 31/713*** *(2006.01)*
***A61K 47/28*** *(2006.01)* ***A61P 9/10*** *(2006.01)*

(52) Cooperative Patent Classification (CPC):
**A61K 31/713; A61K 47/28; A61P 9/10; C12N 15/113**

(86) International application number:
**PCT/CN2021/141665**

(87) International publication number:
**WO 2022/143531 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.12.2020 CN 202011599061**



(71) Applicant: **Suzhou Ribo Life Science Co., Ltd.**
**Kunshan, Jiangsu 215300 (CN)**

(72) Inventors:
- **LIANG, Zicai**
  **Kunshan, Jiangsu 215300 (CN)**
- **ZHANG, Hongyan**
  **Kunshan, Jiangsu 215300 (CN)**
- **GAO, Shan**
  **Kunshan, Jiangsu 215300 (CN)**
- **LI, Jiabin**
  **Kunshan, Jiangsu 215300 (CN)**

(74) Representative: **SSM Sandmair Patentanwälte Rechtsanwalt Partnerschaft mbB**
**Joseph-Wild-Straße 20**
**81829 München (DE)**


Remarks:
The complete document including Reference Table(s) and the Sequence Listing(s) can be downloaded from the EPO website

(54) **NUCLEIC ACID, PHARMACEUTICAL COMPOSITION AND SIRNA CONJUGATE CONTAINING THE NUCLEIC ACID, PREPARATION METHOD THEREFOR, AND USE THEREOF**


(57) The present disclosure provides siRNA inhibiting gene expression of plasma prekallikrein (PKK), and a pharmaceutical composition and an siRNA conjugate containing the siRNA. Each nucleotide in the siRNA is independently a modified or unmodified nucleotide. The siRNA contains a sense strand and an antisense strand. The sense strand contains a nucleotide sequence I which is the same as the nucleotide sequence represented by SEQ ID NO: 1 in terms of length and different from same in no more than three nucleotides. The antisense strand contains a nucleotide sequence II which is the same as the nucleotide sequence represented by SEQ ID NO: 2 in terms of length and different from same in terms of no more than three nucleotides. The siRNA, the pharmaceutical composition, and the siRNA conjugate provided by the present disclosure can effectively treat and/or prevent pathological conditions or diseases caused by the gene expression of the plasma PKK.


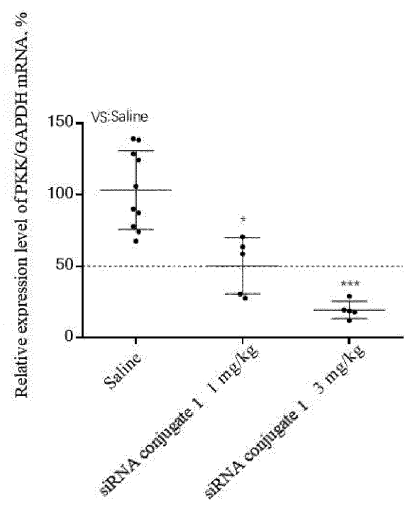


FIG. 1

EP 4 273 245 A1

## Description

### TECHNICAL FIELD

The present disclosure relates to a nucleic acid capable of inhibiting expression of a plasma prekallikrein (PKK) gene, and a pharmaceutical composition and an siRNA conjugate containing the nucleic acid. The present disclosure also relates to a preparation method and use of the nucleic acid, the pharmaceutical composition and the siRNA conjugate.

### BACKGROUND

Bradykinin (BK) is the main regulator of enhancing vascular permeability. Too much BK will enhance the leakage of blood vessels, thus aggravating inflammation. Researches show that a hereditary defect of a C1-esterase inhibitor (C1-INH), which is the main natural inhibitor of BK, will lead to hereditary angioedema (HAE). Patients suffering from rare HAE disease often suffer from acute attack of painful edema caused by unknown inducement, while the attack in throat may be life-threatening.

Prekallikrein (PKK) is a precursor of plasma kallikrein (PK), PKK is transformed into PK under the activation of factor XIIa (FXIIa), and PK cleaves high molecular weight kininogen to release bradykinin into blood vessels. Therefore, the excess of BK can be inhibited at a cellular level by inhibiting the expression of a PKK gene, thus preventing and treating diseases or symptoms such as inflammation caused by excessive BK, especially hereditary angioedema. Small interfering RNA (siRNA), based on the mechanism of RNA interference (RNAi), can inhibit or block the expression of any interested target genes in a sequence-specific way, thus achieving the purpose of treating diseases.

One of the keys to develop siRNA drugs for inhibiting the expression of the PKK gene and treating the hereditary angioedema lies in finding a suitable siRNA and modification thereof, as well as an effective delivery system.

### SUMMARY

The inventors of the present disclosure have surprisingly found that the following siRNA and modification sequence thereof provided by the present disclosure can specifically inhibit the expression of the PKK gene, and the pharmaceutical composition or the siRNA conjugate containing the siRNA can specifically target the liver, thereby inhibiting the expression of the PKK gene in the liver and realizing the treatment or prevention of inflammatory diseases, especially hereditary angioedema, thus completing the present disclosure.

In some embodiments, the present disclosure provides an siRNA capable of inhibiting expression of a PKK gene. The siRNA comprises a sense strand and an antisense strand, each nucleotide in the siRNA is independently a modified or unmodified nucleotide, wherein the sense strand comprises a nucleotide sequence I, and the antisense strand comprises a nucleotide sequence II; the nucleotide sequence I and the nucleotide sequence II are at least partly reverse complementary to form a double-stranded region, and the nucleotide sequence I and the nucleotide sequence II are selected from the sequences shown in the following:

the nucleotide sequence I has the same length as and no more than three nucleotide difference from the nucleotide sequence shown in SEQ ID NO: 1, and the nucleotide sequence II has the same length as and no more than three nucleotide difference from the nucleotide sequence shown in SEQ ID NO: 2:

> 5'-CUGAAUUCCAAAAACCAA$Z_1$-3' (SEQ ID NO: 1);
> 5'-$Z_2$UUGGUUUUUGGAAUUCAG-3' (SEQ ID NO: 2),
> wherein, $Z_1$ is U, $Z_2$ is A, and
> the nucleotide sequence I comprises a nucleotide $Z_3$ at a corresponding site to $Z_1$, the nucleotide sequence II comprises a nucleotide $Z_4$ at a corresponding site to $Z_2$, and $Z_4$ is the first nucleotide from the 5' terminal of the antisense strand.

In some embodiments, the present disclosure provides a pharmaceutical composition, wherein the pharmaceutical composition comprises the siRNA of the present disclosure and a pharmaceutically acceptable carrier.

In some embodiments, the present disclosure provides an siRNA conjugate, wherein the siRNA conjugate comprises the siRNA provided by the present disclosure and a conjugating group conjugated to the siRNA.

In some embodiments, the present disclosure provides the use of the siRNA and/or the pharmaceutical composition and/or the siRNA conjugate of the present disclosure in the manufacture of a medicament for treating and/or preventing an inflammatory disease, especially hereditary angioedema.

In some embodiments, the present disclosure provides a method for treating and/or preventing an inflammatory disease or an embolic disease or a physiological condition, especially hereditary angioedema, wherein the method comprises administering an effective amount of the siRNA and/or the pharmaceutical composition and/or the siRNA

conjugate of the present disclosure to a subject in need.

In some embodiments, the present disclosure provides a method for inhibiting the expression of a PKK gene in a cell, wherein the method comprises contacting an effective amount of the siRNA and/or the pharmaceutical composition and/or the siRNA conjugate of the present disclosure with the cell.

In some embodiments, the present disclosure provides a kit, wherein the kit comprises the siRNA and/or the pharmaceutical composition and/or the siRNA conjugate of the present disclosure.

**Incorporated by Reference**

All publications, patents and patent applications mentioned in this specification are incorporated herein by reference to the same extent as each individual publication, patent and patent application is specifically and individually incorporated herein by reference.

**Advantageous Effects**

The siRNA, the pharmaceutical composition and the siRNA conjugate containing the siRNA provided by the present disclosure have good stability, higher PKK mRNA inhibitory activity and low off-target effect, and/or can significantly treat, prevent or alleviate a pathological condition or disease symptom caused by gene expression of plasma prekallikrein.

For example, at a siRNA concentration of 50 nM, an inhibition percentage of the siRNA of the present disclosure on PKK mRNA in primary hepatocytes of C57BL/6J mice can reach 72.3%. For another example, the siRNA conjugate provided by the present disclosure shows high inhibitory activity on PKK mRNA in mice, and shows certain inhibitory effect on PKK target sequences at different dosages, and the inhibition percentage on PKK mRNA can reach 81.31%. For another example, the inhibition percentage of the siRNA conjugate provided by the present disclosure on PKK mRNA in foot swelling model mice can reach 81.43%.

For another example, the siRNA, the pharmaceutical composition or the siRNA conjugate provided by the present disclosure does not exhibit significant off-target effect. An off-target effect may be, for example, inhibition on normal expression of a gene which is not the target mRNA. It is generally considered that the off-target effect is insignificant, if the binding/inhibition of off-target mRNA expression is at a level of lower than 50%, 40%, 30%, 20%, or 10% of the on-target mRNA effect.

For another example, the siRNA, the pharmaceutical composition or the siRNA conjugate provided by the present disclosure shows a drug effect of obviously inhibiting swelling. By inhibiting the expression of PKK mRNA, the siRNA conjugate provided by the present disclosure has a dose-dependent inhibitory effect on foot swelling of carrageenan-induced mice, and this inhibitory effect on foot swelling is equivalent to or even better than that of positive control drugs. The siRNA conjugate provided by the present disclosure shows high inhibitory activity on PKK mRNA in carrageenan-induced foot swelling model mice, and the inhibition percentage on PKK mRNA can reach more than 60% at different dosages, wherein the inhibition percentage of PKK mRNA reaches 77.47% at a dosage of 3 mg/kg. When the dosage is 9 mg/kg, the inhibition percentage on PKK mRNA can reach 82.43%, which shows obvious dose-dependent inhibition. However, a positive drug indomethacin or icatibant has no significant inhibition on PKK mRNA expression. In terms of drug effect, a maximum swelling degree of foot swelling model mice injected with normal saline reaches 70.6%, while a maximum swelling degree of the mice injected with the siRNA conjugate of the present disclosure is greatly reduced to below 39%, and the time to reach the maximum swelling degree is short, which is only 2 hours, and then the swelling degree is weakened, which indicates that the pain borne by animals is reduced.

The above results show that the siRNA, the pharmaceutical composition and the siRNA conjugate provided by the present disclosure can inhibit the expression of PKK gene, effectively treat and/or prevent an inflammatory disease or an embolic disease or a physiological condition, especially hereditary angioedema and its related symptoms, and have good application prospects.

Other features and advantages of the present disclosure will be described in detail in the detailed description section that follows.

## BRIEF DESCRIPTION OF THE DRAWINGS

Figure 1 is a scatter diagram showing relative expression levels of PKK mRNA in mice (*in vivo*) after administration of different doses of siRNA conjugates.

## DETAILED DESCRIPTION

The specific embodiments of the present disclosure are described in detail as below. It should be understood

that the specific embodiments described herein are only for the purpose of illustration and explanation of the present disclosure and are not intended to limit the present disclosure.

In the present disclosure, PKK mRNA refers to the mRNA with the sequence shown by Genbank registration number NM_008455.4, NM_001318394.1 or NM_001318396.1. Furthermore, unless otherwise stated, the term "target gene" used in the present disclosure refers to a gene capable of transcribing the above PKK mRNA, and the term "target mRNA" refers to the above PKK mRNA.

Definitions

In the context of the present disclosure, unless otherwise specified, capital letters C, G, U, and A indicate the base composition of the nucleotides; the lowercase m indicates that the nucleotide adjacent to the left side of the letter m is a methoxy modified nucleotide; the lowercase f indicates that the nucleotide adjacent to the left side of the letter f is a fluoro modified nucleotide; the lowercase letter s indicates that the two nucleotides adjacent to the left and right of the letter s are linked by a phosphorothioate group; P1 represents that the nucleotide adjacent to the right side of P1 is a 5'-phosphate nucleotide or a 5'-phosphate analogue modified nucleotide, the letter combination VP represents that the nucleotide adjacent to the right side of the letter combination VP is a vinyl phosphate modified nucleotide, the letter combination Ps represents that the nucleotide adjacent to the right side of the letter combination Ps is a phosphorothioate modified nucleotide, and the capital letter P represents that the nucleotide adjacent to the right side of the letter P is a 5'-phosphate nucleotide.

In the context of the present disclosure, the "fluoro modified nucleotide" refers to a nucleotide formed by substituting the hydroxy at the 2' position of the ribose group of the nucleotide with a fluoro, and the "non-fluoro modified nucleotide" refers to a nucleotide formed by substituting the hydroxy at the 2' position of the ribose group of the nucleotide with a non-fluoro group, or a nucleotide analogue. The "nucleotide analogue" refers to a group that can replace a nucleotide in a nucleic acid, while structurally differs from an adenine ribonucleotide, a guanine ribonucleotide, a cytosine ribonucleotide, a uracil ribonucleotide or a thymidine deoxyribonucleotide, such as an isonucleotide, a bridged nucleic acid (BNA) or an acyclic nucleotide. The "methoxy modified nucleotide" refers to a nucleotide formed by substituting the 2'-hydroxy of the ribose group with a methoxy group.

In the context of the present disclosure, expressions "complementary" and "reverse complementary" can be interchangeably used, and have a well-known meaning in the art, namely, in a double-stranded nucleic acid molecule, the bases in one strand are paired complementally with those in the other strand. In DNA, a purine base adenine (A) is always paired with a pyrimidine base thymine (T) (or uracil (U) in RNAs); and a purine base guanine (G) is always paired with a pyrimidine base cytosine (C). Each base pair comprises a purine and a pyrimidine. While adenines in one strand are always paired with thymines (or uracils) in another strand, and guanines are always paired with cytosines, these two strands are considered as being complementary to each other, and the sequence of a strand may be deduced from the sequence of its complementary strand. Correspondingly, in the art a "mispairing" means that in a double-stranded nucleic acid, the bases at corresponding sites are not presented in a manner of being paired complementally.

In the context of the present disclosure, unless otherwise specified, "basically reverse complementary" means that there are no more than 3 base mispairings between two nucleotide sequences. "Substantially reverse complementary" means that there is no more than 1 base mispairing between two nucleotide sequences. "Completely complementary" means that there is no base mispairing between two nucleotide sequences.

In the context of the present disclosure, when a nucleotide sequence has "nucleotide difference" from another nucleotide sequence, the base type of the nucleotide at the same position therebetween are changed. For example, if a nucleotide base in the second sequence is A and the nucleotide base at the same position in the first sequence is U, C, G or T, these two nucleotide sequences are considered as having a nucleotide difference at this position. In some embodiments, if a nucleotide at a position is replaced with an abasic nucleotide or a nucleotide analogue, it is also considered that there is a nucleotide difference at the position.

In the context of the present disclosure, particularly in the description of the method for preparing the siRNA, the composition comprising the siRNA or the siRNA conjugate of the present disclosure, unless otherwise specified, the nucleoside monomer refers to, according to the type and sequence of the nucleotides in the siRNA or siRNA conjugate to be prepared, unmodified or modified RNA phosphoramidites used in a solid phase phosphoramidite synthesis (the RNA phosphoramidites are also called as Nucleoside phosphoramidites elsewhere). Solid phase phosphoramidite synthesis is a well-known method used in RNA synthesis to those skilled in the art. Nucleoside monomers used in the present disclosure are all commercially available.

In the context of the present disclosure, unless otherwise stated, "conjugating" refers to two or more chemical moieties each with specific function being linked to each other via a covalent linkage. Correspondingly, a "conjugate" refers to a compound formed by covalent linkage of the individual chemical moieties. Further, an "siRNA conjugate" represents a compound formed by covalently linking one or more chemical moieties with specific functions to siRNA. The siRNA conjugate should be understood according to the context as the generic term of siRNA conjugates, the

generic term of siRNA conjugates shown in formulae (305) and (307), or the generic term of siRNA conjugates shown in formulae (305), (307) and (308). In the context of the present disclosure, a "conjugating molecule" should be understood as a specific compound capable of being conjugated to an siRNA via reactions, thus finally forming the siRNA conjugate of the present disclosure.

As used herein, a hyphen ("-") that is not between two letters or between two symbols is used to indicate a connection point of a substituent. For example, $-C_1-C_{10}$ alkyl-$NH_2$ is connected through $-C_1-C_{10}$ alkyl.

As used herein, "optional" or "optionally" means that the subsequently described event or condition may or may not occur, and that the description includes instances wherein the event or condition may or may not occur. For example, "optionally substituted" "alkyl" encompasses both "alkyl" and "substituted alkyl" as defined below. In the above or below, substituted groups, such as substituted alkyl, substituted alkoxy, substituted amino, substituted aliphatic groups, substituted heteroaliphatic groups, substituted acyl, substituted aryl or substituted heteroaryl. Unless otherwise specified, a "substituted" group refers to a group in which a hydrogen atom is replaced by one or more substituents. For example, "substituted alkoxy" refers to a group in which one or more hydrogen atoms in the alkoxy are replaced by substituents. It can be understood by those skilled in the art that the compounds that may be used in the application of the present disclosure may contain various substituents, and the substituents can be used in the present disclosure as long as the introduction of the substituents does not affect the function of the present disclosure and can achieve the purpose of the present disclosure. In some embodiments, the substituent is selected from the group consisting of $C_1-C_{10}$ alkyl, $C_6-C_{10}$ aryl, $C_5-C_{10}$ heteroaryl, $C_1-C_{10}$ haloalkyl, $-OC_1-C_{10}$ alkyl, $OC_1-C_{10}$ alkylphenyl, $-C_1-C_{10}$ alkyl-OH, $-OC_1-C_{10}$ haloalkyl, $-SC_1-C_{10}$ alkyl, $-SC_1-C_{10}$ alkylphenyl, $-C_1-C_{10}$ alkyl-SH, $-SC_1-C_{10}$ haloalkyl, halogen substituent, -OH, -SH, $-NH_2$, $-C_1-C_{10}$ alkyl-$NH_2$, $-N(C_1-C_{10}$ alkyl$)(C_1-C_{10}$ alkyl), $-NH(C_1-C_{10}$ alkyl), $-N(C_1-C_{10}$ alkyl$)(C_1-C_{10}$ alkylphenyl), $-NH(C_1-C_{10}$ alkylphenyl), cyano, nitro, $-CO_2H$, $-C(O)O(C_1-C_{10}$ alkyl), $-CON(C_1-C_{10}$ alkyl$)(C_1-C_{10}$ alkyl),$-CONH(C_1-C_{10}$ alkyl), $-CONH_2$, $-NHC(O)(C_1-C_{10}$ alkyl), -NHC(O)(phenyl), $-N(C_1-C_{10}$ alkyl$)C(O)(C_1-C_{10}$ alkyl), $-N(C_1-C_{10}$ alkyl)C(O)(phenyl), $-C(O)C_1-C_{10}$ alkyl, $-C(O)C_1-C_{10}$ alkylphenyl, $-C(O)C_1-C_{10}$ haloalkyl, $-OC(O)C_1-C_{10}$ alkyl, $-SO_2(C_1-C_{10}$ alkyl), $-SO_2$(phenyl), $-SO_2(C_1-C_{10}$ haloalkyl), $-SO_2NH_2$, $-SO_2NH(C_1-C_{10}$ alkyl), $-SO_2NH$(phenyl), $-NHSO_2(C_1-C_{10}$ alkyl), $-NHSO_2$(phenyl) and $-NHSO_2(C_1-C_{10}$ haloalkyl). In some embodiments, the substituent is one of $C_1-C_3$ alkyl, $C_6-C_8$ aryl, $-OC_1-C_3$ alkyl, $-OC_1-C_3$ alkylphenyl, halogen, -OH, $-NH_2$, cyano or nitro. Those skilled in the art would understand, with respect to any group containing one or more substituents, that such groups are not intended to introduce any substitution or substitution patterns that are sterically impractical, synthetically infeasible and/or inherently unstable.

As used herein, "alkyl" refers to straight chain and branched chain having the specified number of carbon atoms, usually 1 to 20 carbon atoms, for example 1 to 10 carbon atoms, such as 1 to 8 or 1 to 6 carbon atoms. For example, $C_1-C_6$ alkyl encompasses both straight and branched chain alkyl of 1 to 6 carbon atoms. When naming an alkyl residue having a specific number of carbon atoms, all branched and straight chain forms having that number of carbon atoms are intended to be encompassed; thus, for example, "butyl" is meant to include n-butyl, sec-butyl, isobutyl and t-butyl; and "propyl" includes n-propyl and isopropyl. Alkylene is a subset of alkyl, referring to the same residues as alkyl, but having two attachment points.

As used herein, "alkenyl" refers to an unsaturated branched or linear alkyl having at least one carbon-carbon double bond which is obtained by removing one hydrogen molecule from two adjacent carbon atoms of the parent alkyl. The group may be in either cis or trans configuration of the double bond. Typical alkenyl groups include, but not limited to, ethenyl; propenyl such as prop-1-en-1-yl, prop-1-en-2-yl, prop-2-en-1-yl (allyl), prop-2-en-2-yl; and butenyl such as but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl, and the like. In certain embodiments, an alkenyl group has 2 to 20 carbon atoms, and in other embodiments, 2 to 10, 2 to 8, or 2 to 6 carbon atoms. Alkenylene is a subset of alkenyl, referring to the same residues as alkenyl, but having two attachment points.

As used herein, "alkynyl" refers to an unsaturated branched or linear alkyl having at least one carbon-carbon triple bond which is obtained by removing two hydrogen molecules from two adjacent carbon atoms of the parent alkyl. Typical alkynyl groups include, but not limited to, ethynyl; propynyl such as prop-1-yn-1-yl, prop-2-yn-1-yl; and butynyl such as but-1-yn-1-yl, but-1-yn-3-yl, but-3-yn-1-yl, and the like. In certain embodiments, an alkynyl group has 2 to 20 carbon atoms, and in other embodiments, 2 to 10, 2 to 8, or 2 to 6 carbon atoms. Alkynylene is a subset of alkynyl, referring to the same residues as alkynyl, but having two attachment points.

As used herein, "alkoxy" refers to an alkyl group of the specified number of carbon atoms attached through an oxygen bridge, such as, methoxy, ethoxy, propoxy, isopropoxy, n-butoxy, sec-butoxy, tert-butoxy, pentyloxy, 2-pentyloxy, isopentyloxy, neopentyloxy, hexyloxy, 2-hexyloxy, 3-hexyloxy, 3-methylpentyloxy, and the like. An alkoxy usually has 1 to 10, 1 to 8, 1 to 6, or 1 to 4 carbon atoms attached through oxygen bridge.

As used herein, "aryl" refers to a group derived from an aromatic monocyclic or multicyclic hydrocarbon ring system by removing a hydrogen atom from a ring carbon atom. The aromatic monocyclic or multicyclic hydrocarbon ring system contains only hydrogen and carbon comprising 6 to 18 carbon carbon atoms, wherein at least one ring in the ring system is fully unsaturated, i.e., containing a cyclic, delocalized $(4n+2)\pi$-electron system in accordance with the Hückel theory. Aryl groups include, but not limited to, phenyl, fluorenyl, naphthyl and the like. Arylene is a subset of aryl,

referring to the same residues as aryl, but having two attachment positions.

As used herein, "cycloalkyl" refers to a nonaromatic carbon ring, usually having 3 to 7 ring carbon atoms. The ring may be saturated or have one or more carbon-carbon double bonds. Examples of the cycloalkyl include cyclopropyl, cyclobutyl, cyclopentyl, cyclopentenyl, cyclohexyl and cyclohexenyl, as well as bridged and caged ring groups such as norbornane.

As used herein, "halo substituent" or "halogenation " refers to fluoro, chloro, bromo, and iodo, and the term "halogen" includes fluorine, chlorine, bromine, or iodine.

As used herein, "haloalkyl" refers to the alkyl as defined above with the specified number of carbon atoms being substituted with one or more halogen atoms, up to the maximum allowable number of halogen atoms. Examples of haloalkyl include, but not limited to, trifluoromethyl, difluoromethyl, 2-fluoroethyl, and pentafluoroethyl.

"Heterocyclyl" refers to a stable 3- to 18-membered non-aromatic ring group that comprises 2-12 carbon atoms and 1-6 heteroatoms selected from nitrogen, oxygen and sulfur. Unless stated otherwise in the description, heterocyclyl is a monocyclic, bicyclic, tricyclic, or tetracyclic ring system, which may include fused or bridged ring systems. The heteroatoms in the heterocyclyl may be optionally oxidized. One or more nitrogen atoms, if present, are optionally quaternized. The heterocyclyl is partially or fully saturated. The heterocyclyl may be linked to the rest of the molecule through any atom of the ring. Examples of such heterocyclyl include, but not limited to, dioxanyl, thienyl[1,3]disulfonyl, decahydroisoquinolyl, imidazolinyl, imidazolidinyl, isothiazolidinyl, isoxazolidinyl, morpholinyl, octahydroindolyl, octahydroisoindolyl, 2-oxapiperazinyl, 2-oxapiperidinyl, 2-oxapyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl.

Various hydroxy protecting groups may be used in the present disclosure. In general, protecting groups render chemical functional groups insensitive to specific reaction conditions, and may be attached to and removed from such functional groups in a molecule without substantially damaging the remainder of the molecule. Representative hydroxy protecting groups are disclosed in Beaucage, et al., Tetrahedron 1992, 48, 2223-2311, and also in Greene and Wuts, Protective Groups in Organic Synthesis, Chapter 2, 2d ed, John Wiley & Sons, New York, 1991, each of which is hereby incorporated by reference in their entirety. In some embodiments, the protecting group is stable under basic conditions but can be removed under acidic conditions. In some embodiments, non-exclusive examples of the hydroxy protecting groups used herein include dimethoxytrityl (DMT), monomethoxytrityl, 9-phenylxanthen-9-yl (Pixyl), and 9-(p-methoxyphenyl)xanthen-9-yl (Mox). In some embodiments, non-exclusive examples of the hydroxy protecting groups used herein include Tr (trityl), MMTr (4-methoxytrityl), DMTr (4,4'-dimethoxytrityl), and TMTr (4,4',4"-trimethoxytrityl).

The term "subject", as used herein, refers to any animal, e.g., mammal or marsupial. The subject of the present disclosure includes, but not limited to, human, non-human primate (e.g., rhesus or other kinds of macaque), mouse, pig, horse, donkey, cow, sheep, rat, rabbit and any kind of poultry.

As used herein, "treatment", "alleviating" or "improving" may be used interchangeably herein. These terms refer to methods for obtaining advantageous or desired result, including but not limited to, therapeutic benefit. "Therapeutic benefit" means eradication or improvement of potential disorder to be treated. Moreover, the therapeutic benefit is achieved by eradicating or ameliorating one or more of physiological symptoms associated with the potential disorder such that an improvement is observed in the subject, notwithstanding that the subject may still be afflicted with the potential disorder.

As used herein, "preventing" and "prevention" may be used interchangeably. These terms refer to methods for obtaining beneficial or expected results, including but not limited to prophylactic benefits. For obtaining the "prophylactic benefit", the siRNA, the siRNA conjugate or the pharmaceutical composition may be administered to the subject at risk of developing a particular disease, or to the subject reporting one or more physiological symptoms of a disease, even though the diagnosis of this disease may not have been made.

## siRNA

In one aspect, the present disclosure provides an siRNA capable of inhibiting the expression of a PKK gene.

The siRNA of the present disclosure comprises nucleotides as basic structural units. It is well-known to those skilled in the art that the nucleotide comprises a phosphate group, a ribose group and a base. Detailed illustrations relating to such groups are omitted herein.

The siRNA comprises a sense strand and an antisense strand. Each nucleotide in the siRNA is independently a modified or unmodified nucleotide, wherein the sense strand comprises a nucleotide sequence I, the antisense strand comprises a nucleotide sequence II, and the nucleotide sequence I and the nucleotide sequence II are at least partly reverse complementary to form a double-stranded region, wherein the nucleotide sequence I has the same length as and no more than three nucleotide differences from the nucleotide sequence shown in SEQ ID NO: 1; and the nucleotide sequence II has the same length as and no more than three nucleotide differences from the nucleotide sequence shown in SEQ ID NO: 2:

5'-CUGAAUUCCAAAAACCAA$Z_1$-3' (SEQ ID NO: 1);
5'-$Z_2$UUGGUUUUUGGAAUUCAG-3' (SEQ ID NO: 2),
wherein, $Z_1$ is U, $Z_2$ is A, and
the nucleotide sequence I comprises a nucleotide $Z_3$ at a corresponding site to $Z_1$, the nucleotide sequence II comprises a nucleotide $Z_4$ at a corresponding site to $Z_2$, and $Z_4$ is the first nucleotide from the 5' terminal of the antisense strand.

In this context, the term "corresponding site" means being at the same site in the nucleotide sequence by counting from the same terminal of the nucleotide sequence. For example, the first nucleotide at the 3' terminal of the nucleotide sequence I is a nucleotide at the corresponding site to the first nucleotide at the 3' terminal of SEQ ID NO: 1.
In some embodiments, the sense strand exclusively comprises the nucleotide sequence I, and the antisense strand exclusively comprises the nucleotide sequence II.
In some embodiments, the nucleotide sequence I has no more than one nucleotide difference from the nucleotide sequence shown in SEQ ID NO: 1, and/or the nucleotide sequence II has no more than one nucleotide difference from the nucleotide sequence shown in SEQ ID NO: 2.
In some embodiments, the nucleotide difference between the nucleotide sequence II and the nucleotide sequence shown in SEQ ID NO: 2 comprises a difference at the site of $Z_4$, and $Z_4$ is selected from U, C or G. In some embodiments, the nucleotide difference is a difference at the site of $Z_4$, and $Z_4$ is selected from U, C or G. In some embodiments, $Z_3$ is a nucleotide complementary to $Z_4$. The siRNAs having the above nucleotide differences have higher ability to inhibit the target mRNA, and these siRNAs comprising nucleotide differences are also within the protection scope of the present disclosure.
In some embodiments, the nucleotide sequence I is basically reverse complementary, substantially reverse complementary, or completely reverse complementary to the nucleotide sequence II. The basically reverse complementary refers to no more than three base mispairings between two nucleotide sequences; the substantially reverse complementary refers to no more than one base mispairing between two nucleotide sequences; and the completely reverse complementary refers to no base mispairing between two nucleotide sequences.
In some embodiments, the nucleotide sequence I is the nucleotide sequence shown in SEQ ID NO: 3, and the nucleotide sequence II is the nucleotide sequence shown in SEQ ID NO: 4:

5'-CUGAAUUCCAAAAACCAA$Z_3$-3' (SEQ ID NO: 3);
5'-$Z_4$UUGGUUUUUGGAAUUCAG-3' (SEQ ID NO: 4),
wherein, $Z_4$ is the first nucleotide from 5' terminal of the antisense strand; $Z_3$ is selected from A, U, G or C; and $Z_4$ is a nucleotide complementary to $Z_3$; and in some embodiments, $Z_3$ is U, and $Z_4$ is A; and
moreover, the lengths of the sense strand and the antisense strand are the same or different, the length of the sense strand is 19-23 nucleotides, and the length of the antisense strand is 19-26 nucleotides. In this way, a length ratio of the sense strand to the antisense strand of the siRNA provided by the present disclosure may be 19/19, 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25 or 23/26. In some embodiments, the length ratio of the sense strand to the antisense strand of the siRNA is 19/21, 21/23 or 23/25.

In some embodiments, the sense strand further comprises a nucleotide sequence III, the antisense strand further comprises a nucleotide sequence IV, and the nucleotide sequence III and the nucleotide sequence IV each has a length of 1-4 nucleotides; the nucleotide sequence III has the same length and is substantially reverse complementary or completely reverse complementary to the nucleotide sequence IV; the nucleotide sequence III is linked to the 5' terminal of the nucleotide sequence I, and the nucleotide sequence IV is linked to the 3' terminal of the nucleotide sequence II. In some embodiments, the nucleotide sequence IV is substantially reverse complementary or completely reverse complementary to the second nucleotide sequence, and the second nucleotide sequence refers to the nucleotide sequence adjacent to the 5' terminal of the nucleotide sequence represented by SEQ ID NO: 1 in the target mRNA and having the same length as the nucleotide sequence IV.
In some embodiments, in the direction from the 5' terminal to the 3' terminal, the nucleotide sequence III and the nucleotide sequence IV both have a length of one nucleotide, the base of the nucleotide sequence III is A, and the base of the nucleotide sequence IV is U; in this case, the length ratio of the sense strand to the antisense strand is 20/20; or, the nucleotide sequences III and IV both have a length of two nucleotides, and in the direction from the 5' terminal to the 3' terminal, the base composition of the nucleotide sequence III is UA, and the base composition of the nucleotide sequence IV is UA; in this case, the length ratio of the sense strand to the antisense strand is 21/21; or, the nucleotide sequences III and IV both have a length of three nucleotides, and in the direction from the 5' terminal to the 3' terminal, the base composition of the nucleotide sequence III is AUA, and the base composition of the nucleotide

sequence IV is UAU; in this case, the length ratio of the sense strand to the antisense strand is 22/22; or, the nucleotide sequences III and IV both have a length of four nucleotides, and in the direction from the 5' terminal to the 3' terminal, the base composition of the nucleotide sequence III is UAUA, and the base composition of the nucleotide sequence IV is UAUA; in this case, the length ratio of the sense strand to the antisense strand is 23/23. In some embodiments, the nucleotide sequence III and the nucleotide sequence IV have a length of two nucleotides, and in the direction from the 5' terminal to the 3' terminal, the base composition of the nucleotide sequence III is UA, and the base composition of the nucleotide sequence IV is UA; in this case, the length ratio of the sense strand to the antisense strand is 21/21.

In some embodiments, the nucleotide sequence III is completely reverse complementary to the nucleotide sequence IV. Thus, if the bases of the nucleotide sequence III are provided, the bases of the nucleotide sequence IV are also determined.

In some embodiments, the antisense strand further comprises a nucleotide sequence V, the nucleotide sequence V has a length of 1-3 nucleotides and is linked to the 3' terminal of the antisense strand, thereby constituting a 3' overhang of the antisense strand. As such, the length ratio of the sense strand to the antisense strand in the siRNA of the present disclosure may be 19/20, 19/21, 19/22, 20/21, 20/22, 20/23, 21/22, 21/23, 21/24, 22/23, 22/24, 22/25, 23/24, 23/25, or 23/26. In some embodiments, the nucleotide sequence V has a length of 2 nucleotides. As such, the length ratio of the sense strand to the antisense strand in the siRNA of the present disclosure may be 19/21, 21/23 or 23/25.

Each nucleotide in the nucleotide sequence V may be any nucleotide. In order to facilitate synthesis and save synthesis cost, the nucleotide sequence V is 2 continuous thymidine deoxyribonucleotides (dTdT) or 2 continuous uracil ribonucleotides (UU); or, in order to improve the affinity of the antisense strand of the siRNA to the target mRNA, the nucleotide sequence V is complementary to the nucleotides at the corresponding site of the target mRNA. Therefore, in some embodiments, the length ratio of the sense strand to the antisense strand of the siRNA of the present disclosure is 19/21 or 21/23. In this case, the siRNA of the present disclosure has better silencing activity against target mRNA.

The nucleotide at the corresponding site of the target mRNA refers to the nucleotide or nucleotide sequence adjacent to a segment of nucleotide sequence of the target mRNA at the 5' terminal. This segment of nucleotide sequence of the target mRNA is substantially reverse complementary or completely reverse complementary to the nucleotide sequence II, or, is a segment of nucleotide sequence which is substantially reverse complementary or completely reverse complementary to the nucleotide sequence formed by the nucleotide sequence II and the nucleotide sequence IV.

In some embodiments, the sense strand of the siRNA comprises the nucleotide sequence shown in SEQ ID NO: 5, and the antisense strand of the siRNA comprises the nucleotide sequence shown in SEQ ID NO: 6:

5'-CUGAAUUCCAAAAACCAA$Z_3$-3' (SEQ ID NO: 5);
5'-$Z_4$UUGGUUUUUGGAAUUCAGUA-3' (SEQ ID NO: 6);
or, the sense strand of the siRNA comprises the nucleotide sequence shown in SEQ ID NO: 7, and the antisense strand of the siRNA comprises the nucleotide sequence shown in SEQ ID NO: 8;
5'-UACUGAAUUCCAAAAACCAA$Z_3$-3' (SEQ ID NO: 7);
5'-$Z_4$UUGGUUUUUGGAAUUCAGUAUA-3'(SEQ ID NO: 8);
wherein, $Z_4$ is the first nucleotide from 5' terminal of the antisense strand; $Z_3$ is selected from A, U, G or C; and $Z_3$ is a nucleotide complementary to $Z_4$.

In some embodiments, the siRNA of the present disclosure is siPKKa1 and siPKKa2 listed in Table 1.

As described above, the nucleotides in the siRNA of the present disclosure are each independently modified or unmodified nucleotides. In some embodiments, each nucleotide in the siRNA of the present disclosure is an unmodified nucleotide. In some embodiments, some or all nucleotides in the siRNA of the present disclosure are modified nucleotides. Such modifications on the nucleotides would not cause significant decrease or loss of the function of the siRNA of the present disclosure to inhibit the expression of PKK genes.

In some embodiments, the siRNA of the present disclosure comprises at least one modified nucleotide. In the context of the present disclosure, the term "modified nucleotide" employed herein refers to a nucleotide formed by substituting the hydroxy at the 2'-position of the ribose group of a nucleotide with other group, or a nucleotide analogue, or a nucleotide with modified base. Such modified nucleotides would not cause significant decrease or loss of the function of the siRNA to inhibit the expression of genes. For example, the modified nucleotides disclosed in J.K. Watts, G. F. Deleavey and M. J.Damha, Chemically Modified siRNA: tools and applications. Drug Discov Today, 2008.13(19-20): p. 842 -855 may be selected.

In some embodiments, at least one nucleotide in the sense strand or the antisense strand of the siRNA provided by the present disclosure is a modified nucleotide, and/or at least one phosphoester group is a phosphoester group with modified group. In other words, at least part of the phosphoester groups and/or ribose groups in phosphate-sugar backbone of at least one single strand in the sense strand and the antisense strand are phosphoester groups with modified group and/or ribose groups with modified group.

In some embodiments, all nucleotides in the sense strand and/or the antisense strand are modified nucleotides.

In some embodiments, each nucleotide in the sense strand and the antisense strand of the siRNA provided by the present disclosure is independently a fluoro modified nucleotide or a non-fluoro modified nucleotide.

The inventors of the present disclosure have surprisingly found that the siRNA of the present disclosure has achieved a high degree of balance between the stability in plasma and the gene silencing efficiency in animal experiments.

In some embodiments, the fluoro modified nucleotides are located in the nucleotide sequence I and the nucleotide sequence II; and in the direction from 5' terminal to 3' terminal, at least the nucleotides at positions 7, 8 and 9 of the nucleotide sequence I are fluoro modified nucleotides; and in the direction from 5' terminal to 3' terminal, at least the nucleotides at positions 2, 6, 14 and 16 of the nucleotide sequence II are fluoro modified nucleotides.

In some embodiments, the fluoro modified nucleotides are located in the nucleotide sequence I and the nucleotide sequence II; no more than 5 fluoro modified nucleotides are present in the nucleotide sequence I, and in the direction from 5' terminal to 3' terminal, at least the nucleotides at positions 7, 8 and 9 in the nucleotide sequence I are fluoro modified nucleotides; no more than 7 fluoro modified nucleotides are present in the nucleotide sequence II, and at least the nucleotides at positions 2, 6, 14 and 16 in the nucleotide sequence II are fluoro modified nucleotides.

In some embodiments, in the direction from 5' terminal to 3' terminal, the nucleotides at positions 7, 8 and 9 or at positions 5, 7, 8 and 9 of the nucleotide sequence I in the sense strand are fluoro modified nucleotides, and the nucleotides at the rest of positions in the sense strand are non-fluoro modified nucleotides; and in the direction from 5' terminal to 3' terminal, the nucleotides at positions 2, 6, 14 and 16 or at positions 2, 6, 8, 9, 14 and 16 of the nucleotide sequence II in the antisense strand are fluoro modified nucleotides, and the nucleotides at the rest of positions in the antisense strand are non-fluoro modified nucleotides.

In the context of the present disclosure, a "fluoro modified nucleotide" refers to a nucleotide which is formed by substituting the hydroxy at the 2'-position of the ribose group of a nucleotide with fluoro, which has a structure as shown by Formula (7). A "non-fluoro modified nucleotide", refers to a nucleotide which is formed by substituting the hydroxy at the 2'-position of the ribose group of a nucleotide with a non-fluoro group, or a nucleotide analogue. In some embodiments, each non-fluoro modified nucleotide is independently selected from a nucleotide formed by substituting the hydroxy at the 2'-position of the ribose group of the nucleotide with the non-fluoro group, or the nucleotide analogue.

These nucleotides formed by substituting the hydroxy at 2'-position of the ribose group with the non-fluoro group are well-known to those skilled in the art, and these nucleotides may be selected from one of a 2'-alkoxy modified nucleotide, a 2'-substituted alkoxy modified nucleotide, a 2'-alkyl modified nucleotide, a 2'-substituted alkyl modified nucleotide, a 2'-amino modified nucleotide, a 2'-substituted amino modified nucleotide and a 2'-deoxy nucleotide.

In some embodiments, the 2'-alkoxy modified nucleotide is a methoxy modified nucleotide (2'-OMe), as shown by Formula (8). In some embodiments, the 2'-substituted alkoxy modified nucleotide is, for example, a 2'-O-methoxyethoxy modified nucleotide (2'-MOE) as shown by Formula (9). In some embodiments, the 2'-amino modified nucleotide ($2'-NH_2$) is as shown by Formula (10). In some embodiments, the 2'-deoxy nucleotide (DNA) is as shown by Formula (11):

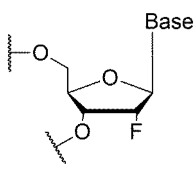
Formula (7),

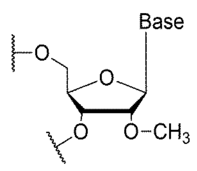
Formula (8),

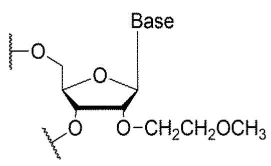
Formula (9),

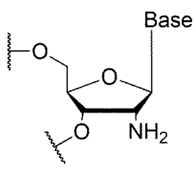
Formula (10),

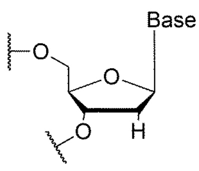
Formula (11).

The nucleotide analogue refers to a group that can replace a nucleotide in a nucleic acid, while structurally differs from an adenine ribonucleotide, a guanine ribonucleotide, a cytosine ribonucleotide, a uracil ribonucleotide or a thymidine deoxyribonucleotide. In some embodiments, the nucleotide analogue may be an isonucleotide, a bridged nucleic acid (referred to as BNA) or an acyclic nucleotide.

The BNA is a nucleotide that is constrained or is not accessible. The BNA may contain a 5-membered ring, 6-membered ring or 7-membered ring bridged structure with a "fixed" C3'-endo sugar puckering. The bridge is typically incorporated at the 2'- and 4'-position of the ribose to provide a 2',4'-BNA nucleotide. In some embodiments, the BNA may be an LNA, an ENA, a cET BNA, etc., wherein the LNA is as shown by Formula (12), the ENA is as shown by Formula (13) and the cET BNA is as shown by Formula (14):

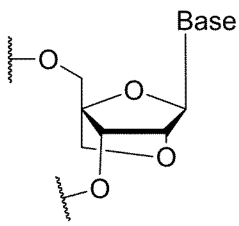 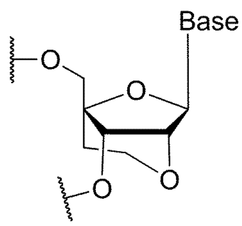 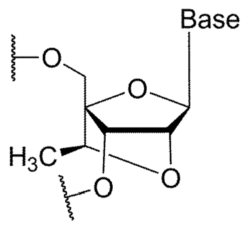

Formula (12), Formula (13), Formula (14).

An acyclic nucleotide is a nucleotide in which a sugar ring is opened. In some embodiments, the acyclic nucleotide may be an unlocked nucleic acid (UNA) or a glycerol nucleic acid (GNA), wherein the UNA is as shown by Formula (15), and the GNA is as shown by Formula (16):

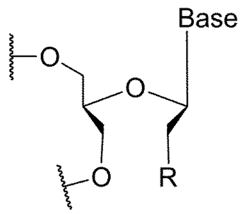 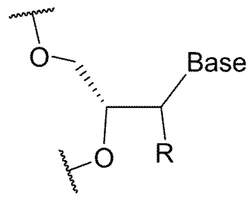

Formula (15), Formula (16).

In the Formula (15) and the Formula (16), R is selected from H, OH or alkoxy (O-alkyl).

An isonucleotide is a compound which is formed by altering the position of the base on the ribose ring in a nucleotide. In some embodiments, the isonucleotide may be a compound in which the base is transferred from l'-position to 2'-position or 3'-position on the ribose ring, as shown by Formula (17) or (18).

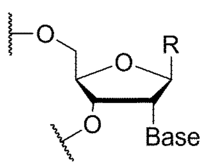 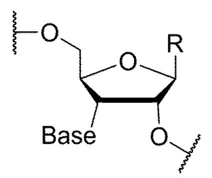

Formula (17), Formula (18).

In the compounds as shown by the Formula (17) and Formula (18) above, Base represents a nucleic acid base, such as A, U, G, C or T; and R is selected from H, OH, F or a non-fluoro group described above.

In some embodiments, the nucleotide analogue is selected from one of an isonucleotide, an LNA, an ENA, a cET, a UNA and a GNA. In some embodiments, each non-fluoro modified nucleotide is a methoxy modified nucleotide. In the context of the present disclosure, the methoxy modified nucleotide refers to a nucleotide formed by substituting the 2'-hydroxy of the ribose group with a methoxy group.

In the context of the present disclosure, a "fluoro modified nucleotide", a "2'-fluoro modified nucleotide", a "nucleotide in which the 2'-hydroxy of the ribose group is substituted with fluoro" and a "nucleotide with 2'-fluororibosyl" have the same meaning, referring to the compound formed by substituting the 2'-hydroxy of the nucleotide with fluoro, having a structure as shown by Formula (7). A "methoxy modified nucleotide", a "2'-methoxy modified nucleotide", a "nucleotide in which the 2'-hydroxy of the ribose group is substituted with methoxy" and a "nucleotide with 2'-methoxyribosyl" have the same meaning, referring to the compound formed by substituting the 2'-hydroxy of the ribose group of the nucleotide with methoxy, having a structure as shown by Formula (8).

In some embodiments, the siRNA of the present disclosure is an siRNA with the following modifications: in the direction from 5' terminal to 3' terminal, the nucleotides at positions 7, 8 and 9 or at positions 5, 7, 8 and 9 of the nucleotide sequence I in the sense strand are fluoro modified nucleotides, and the nucleotides at the rest of positions in the sense

strand are methoxy modified nucleotides; and the nucleotides at positions 2, 6, 14 and 16 or at positions 2, 6, 8, 9, 14 and 16 of the nucleotide sequence II in the antisense strand are fluoro modified nucleotides, and the nucleotides at the rest of positions in the antisense strand are methoxy modified nucleotides.

In some embodiments, the siRNA of the present disclosure is an siRNA with the following modifications: in the direction from 5' terminal to 3' terminal, the nucleotides at positions 5, 7, 8 and 9 of the nucleotide sequence I in the sense strand of the siRNA are fluoro modified nucleotides, and the nucleotides at the rest of positions in the sense strand of the siRNA are methoxy modified nucleotides; and, in the direction from 5' terminal to 3' terminal, the nucleotides at positions 2, 6, 8, 9, 14 and 16 of the nucleotide sequence II in the antisense strand of the siRNA are fluoro modified nucleotides, and the nucleotides at the rest of positions in the antisense strand of the siRNA are methoxy modified nucleotides;

or, in the direction from 5' terminal to 3' terminal, the nucleotides at positions 5, 7, 8 and 9 of the nucleotide sequence I in the sense strand of the siRNA are fluoro modified nucleotides, and the nucleotides at the rest of positions in the sense strand of the siRNA are methoxy modified nucleotides; and, in the direction from 5' terminal to 3' terminal, the nucleotides at positions 2, 6, 14 and 16 of the nucleotide sequence II in the antisense strand of the siRNA are fluoro modified nucleotides, and the nucleotides at the rest of positions in the antisense strand of the siRNA are methoxy modified nucleotides;

or, in the direction from 5' terminal to 3' terminal, the nucleotides at positions 7, 8 and 9 of the nucleotide sequence I in the sense strand of the siRNA are fluoro modified nucleotides, and the nucleotides at the rest of positions in the sense strand of the siRNA are methoxy modified nucleotides; and, in the direction from 5' terminal to 3' terminal, the nucleotides at positions 2, 6, 14 and 16 of the nucleotide sequence II in the antisense strand of the siRNA are fluoro modified nucleotides, and the nucleotides at the rest of positions in the antisense strand of the siRNA are methoxy modified nucleotides.

In some embodiments, the siRNA provided by the present disclosure is any one of siPKKa1-M1, siPKKa1-M2, siPKKa1-M3, siPKKa2-M1, siPKKa2-M2 and siPKKa2-M3 listed in Table 1.

The siRNAs with the above modifications are not only low-cost, but also allow the ribonucleases in the blood to be less liable to cleaving the nucleic acid so as to increase the stability of the nucleic acid and enable the nucleic acid to have stronger resistance against nuclease hydrolysis. Meanwhile, the modified siRNAs above have higher activity of inhibiting the target mRNA.

In some embodiments, at least part of the phosphoester groups in phosphate-sugar backbone of at least one single strand in the sense strand and the antisense strand of the siRNA provided by the present disclosure are phosphoester groups with modified group. In some embodiments, the phosphoester group with modified group is a phosphorothioate group formed by substituting at least one oxygen atom in a phosphodiester bond in the phosphoester group with a sulfur atom; and in some embodiments, the phosphoester group with modified group is a phosphorothioate group having a structure as shown by Formula (1):

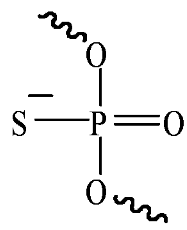

Formula (1).

This modification can stabilize the double-stranded structure of the siRNA, thereby maintaining high specificity and high affinity of base pairing.

In some embodiments, in the siRNA provided by the present disclosure, a phosphorothioate linkage exists in at least one of the group consisiting of the following positions: the position between the first nucleotide and second nucleotides at either terminal of the sense strand or antisense strand; the position between the second and third nucleotides at either terminal of the sense strand or antisense strand; or any combination thereof. In some embodiments, a phosphorothioate linkage exists at all the above positions except for 5' terminal of the sense strand. In some embodiments, a phosphorothioate linkage exists at all the above positions except for 3' terminal of the sense strand. In some embodiments, a phosphorothioate linkage exists in at least one of the following positions:

the position between the first nucleotide and the second nucleotide at 5' terminal of the sense strand;

the position between the second nucleotide and the third nucleotide at 5' terminal of the sense strand;

the position between the first nucleotide and the second nucleotide at 3' terminal of the sense strand;

the position between the second nucleotide and the third nucleotide at 3' terminal of the sense strand;

the position between the first nucleotide and the second nucleotide at 5' terminal of the antisense strand;

the position between the second nucleotide and the third nucleotide at 5' terminal of the antisense strand;

the position between the first nucleotide and the second nucleotide at 3' terminal of the antisense strand; and

the position between the second nucleotide and the third nucleotide at 3' terminal of the antisense strand.

In some embodiments, the siRNA provided by the present disclosure is any one of siPKKa1-M1S, siPKKa1-M2S, siPKKa1-M3S, siPKKa2-M1S, siPKKa2-M2S and siPKKa2-M3S listed in Table 1.

In some embodiments, the 5'-terminal nucleotide in the antisense strand of the siRNA is a 5'-phosphate nucleotide or a 5'-phosphate analogue modified nucleotide.

Common types of the 5'-phosphate nucleotides or 5'-phosphate analogue modified nucleotides are well known to those skilled in the art; for example, the 5'-phosphate nucleotides may have the following structure:

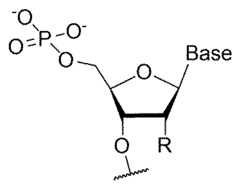

Formula (2).

For another example, Anastasia Khvorova and Jonathan K. Watts, The chemical evolution of oligonucleotide therapies of clinical utility. Nature Biotechnology, 2017, 35(3): 238-248, disclose the following four 5'-phosphate analogue modified nucleotides:

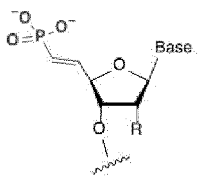

Formula (3)

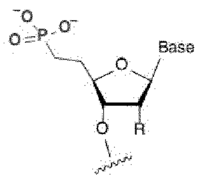

Formula (4)

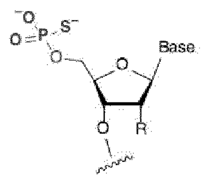

Formula (5)

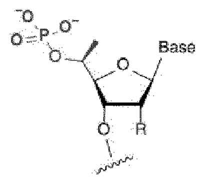

Formula (6),

wherein, R is selected from H, OH, methoxy or F; and Base represents a nucleic acid base selected from A, U, C, G, or T.

In some embodiments, the 5'-phosphate nucleotide is a nucleotide with 5'-phosphate modification as shown by Formula (2); the 5'-phosphate analogue modified nucleotide is a nucleotide with 5'-(E)-vinylphosphonate (E-VP) modification as shown by Formula (3) or a phosphorothioate modified nucleotide as shown by Formula (5).

In some embodiments, the siRNA provided by the present disclosure is any one of siPKKa1-M1P1, siPKKa1-M2P1, siPKKa 1- M3P 1, siPKKa2-M1P1, siPKKa2-M2P1, siPKKa2-M3P1, siPKKa1-M1SP1, siPKKa 1- M2SP 1, siPKKa1-M3SP1, siPKKa2-M1SP1, siPKKa2-M2SP1 and siPKKa2-M3SP1 listed in Table 1.

The inventors of the present disclosure have surprisingly found that the siRNA provided by the present disclosure not only has significantly enhanced plasma and lysosomal stability, but also has higher inhibitory activity of target mRNA.

The siRNA provided by the present disclosure can be obtained by conventional methods for preparing siRNAs in the art (e.g., solid phase synthesis and liquid phase synthesis methods). Wherein, commercial customization services have already been available for solid phase synthesis. Modified nucleotide groups may be introduced into the siRNA of the present disclosure by using a nucleotide monomer having a corresponding modification, wherein the methods for preparing the nucleotide monomer having the corresponding modification and the methods for introducing the modified nucleotide group into the siRNA are also well-known to those skilled in the art.

Pharmaceutical composition

In another aspect, the present disclosure provides a pharmaceutical composition, wherein the pharmaceutical composition comprises the siRNA described above as an active ingredient, and a pharmaceutically acceptable carrier.

The pharmaceutically acceptable carrier may be a carrier conventionally used in the field of siRNA administration, for example, but not limited to, one or more of magnetic nanoparticles (such as $Fe_3O_4$ or $Fe_2O_3$-based nanoparticle), carbon nanotubes, mesoporous silicon, calcium phosphate nanoparticles, polyethylenimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid) copolymer (PLGA), poly(2-aminoethyl ethylene phosphate) (PPEEA), poly(2-dimethylaminoethyl methacrylate) (PDMAEMA), and derivatives thereof.

In some embodiments, in the pharmaceutical composition, there are no special requirements for the contents of the siRNA and the pharmaceutically acceptable carrier. In some embodiments, the weight ratio of the siRNA to the pharmaceutically acceptable carrier is 1 :(1-500), and in some embodiments, the weight ratio above is 1 :(1-50).

In some embodiments, the pharmaceutical composition may also comprise other pharmaceutically acceptable excipients, which may be one or more of various conventional formulations or compounds in the art. For example, other pharmaceutically acceptable excipients may comprise at least one of a pH buffer solution, a protective agent and an osmotic pressure regulator.

The pH buffer solution may be a tris(hydroxymethyl) aminomethane hydrochloride buffer solution with a pH of 7.5-8.5, and/or a phosphate buffer solution with a pH of 5.5-8.5, for example a phosphate buffer solution with a pH of 5.5-8.5.

The protective agent may be at least one of inositol, sorbitol, sucrose, trehalose, mannose, maltose, lactose, and glucose. The content of the protective agent may be from 0.01 wt% to 30 wt% on the basis of the total weight of the pharmaceutical composition.

The osmotic pressure regulator may be sodium chloride and/or potassium chloride. The content of the osmotic pressure regulator allows an osmotic pressure of the pharmaceutical composition to be 200-700 milliosmol/kg (mOsm/kg). Depending on the desired osmotic pressure, those skilled in the art can readily determine the content of the osmotic pressure regulator.

In some embodiments, the pharmaceutical composition may be a liquid formulation, for example, an injection solution; or a lyophilized powder for injection, which is mixed with a liquid excipient to form a liquid formulation upon administration. The liquid formulation may be administered by, but not limited to, subcutaneous, intramuscular or intravenous injection routes, and also may be administered to, but not limited to, lung by spray, or other organs (such as liver) via lung by spray. In some embodiments, the pharmaceutical composition is administered by intravenous injection.

In some embodiments, the pharmaceutical composition may be in the form of a liposome formulation. In some embodiments, the pharmaceutically acceptable carrier used in the liposome formulation comprises an amine-containing transfection compound (hereinafter also referred to as an organic amine), a helper lipid and/or a pegylated lipid. The organic amine, the helper lipid and the pegylated lipid may be respectively selected from one or more of the amine-containing transfection compounds or the pharmaceutically acceptable salts or derivatives thereof, the helper lipids and the pegylated lipids as described in Chinese patent application CN103380113A (which is incorporated herein by reference in its entirety).

In some embodiments, the organic amine may be a compound as shown by Formula (201) as described in CN103380113A or a pharmaceutically acceptable salt thereof:

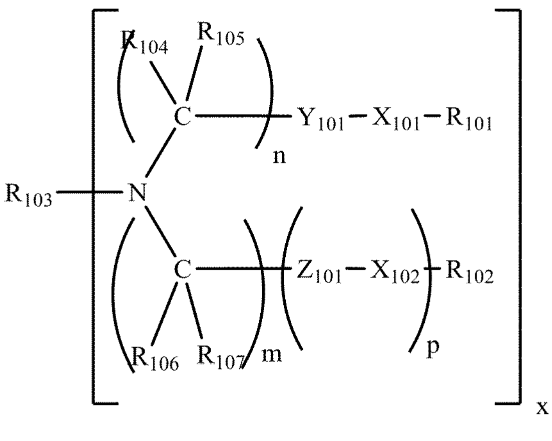

Formula (201),

wherein:

each $X_{101}$ or $X_{102}$ is independently selected from O, S, N-A or C-A, wherein A is hydrogen or a $C_1$-$C_{20}$ hydrocarbon chain;

each $Y_{101}$ or $Z_{101}$ is independently selected from C=O, C=S, S=O, CH-OH or $SO_2$;

each $R_{101}$, $R_{102}$, $R_{103}$, $R_{104}$, $R_{105}$, $R_{106}$ or $R_{107}$ is independently hydrogen; a cyclic or acyclic, substituted or unsubstituted, branched or linear aliphatic group; a cyclic or acyclic, substituted or unsubstituted, branched or linear heteroaliphatic group; a substituted or unsubstituted, branched or linear acyl group; a substituted or unsubstituted, branched or linear aryl, or a substituted or unsubstituted, branched or linear heteroaryl;

x is an integer of 1-10;

n is an integer of 1-3, m is an integer of 0-20, and p is 0 or 1, wherein if m=p=0, then $R_{102}$ is hydrogen, and if at least one of n or m is 2, then $R_{103}$ and the nitrogen in Formula (201) form a structure as shown by Formula (202) or (203):

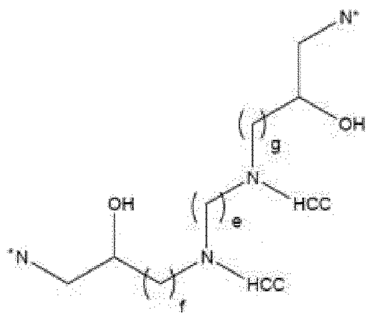
Formula (202),
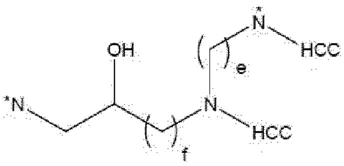
Formula (203);

wherein g, e and f are each independently an integer of 1-6, "HCC" represents a hydrocarbon chain, and each *N represents a nitrogen atom shown in Formula (201).

In some embodiments, $R_{103}$ is a polyamine. In other embodiments, $R_{103}$ is a ketal. In some embodiments, each of $R_{101}$ and $R_{102}$ in the Formula (201) is independently any substituted or unsubstituted, branched or linear alkyl or alkenyl, wherein the alkyl or alkenyl has 3 to about 20 carbon atoms (such as 8 to about 18 carbon atoms) and 0 to 4 double bonds (such as 0 to 2 double bonds).

In some embodiments, if each of n and m is independently 1 or 3, $R_{103}$ may be any of the following Formulae (204)-(213):

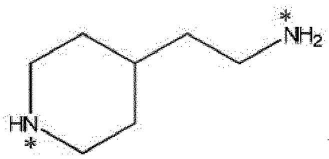
Formula (204),
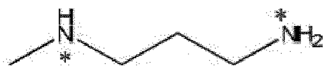
Formula (205),

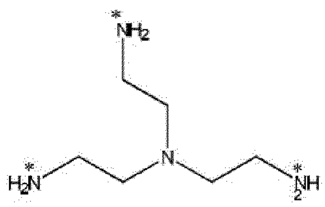
Formula (206),
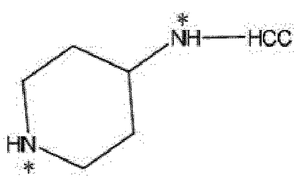
Formula (207),

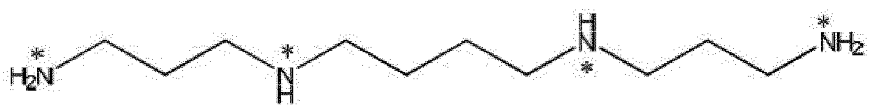
Formula (208),

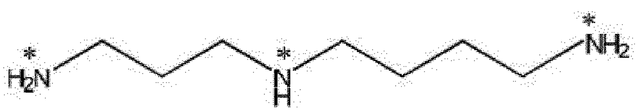
Formula (209),

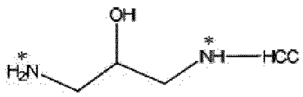 Formula (210), 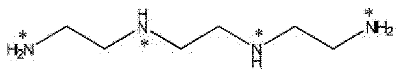 Formula (211),

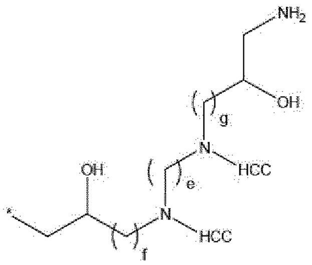 Formula (212) and 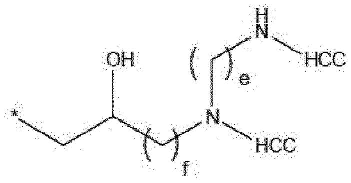 Formula (213);

wherein, in Formula (204) to Formula (213), each of g, e and f is independently an integer of 1-6; each "HCC" represents a hydrocarbon chain, and each * represents a potential attachment point of $R_{103}$ to the nitrogen atom in Formula (201), wherein each H at any * position may be replaced to realize the attachment to the nitrogen atom in Formula (201).

Wherein, the compound as shown by (201) may be prepared as described in CN103380113A.

In some embodiments, the organic amine may be an organic amine as shown by Formula (214) and/or an organic amine as shown by Formula (215):

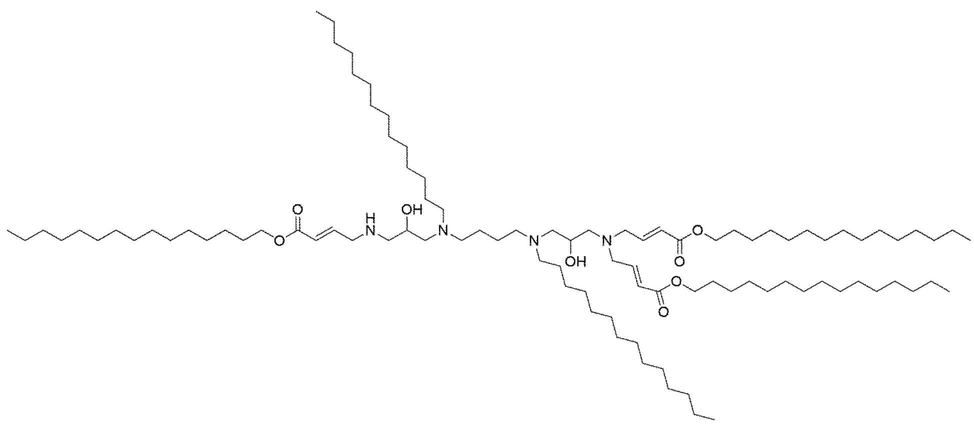 Formula (214),

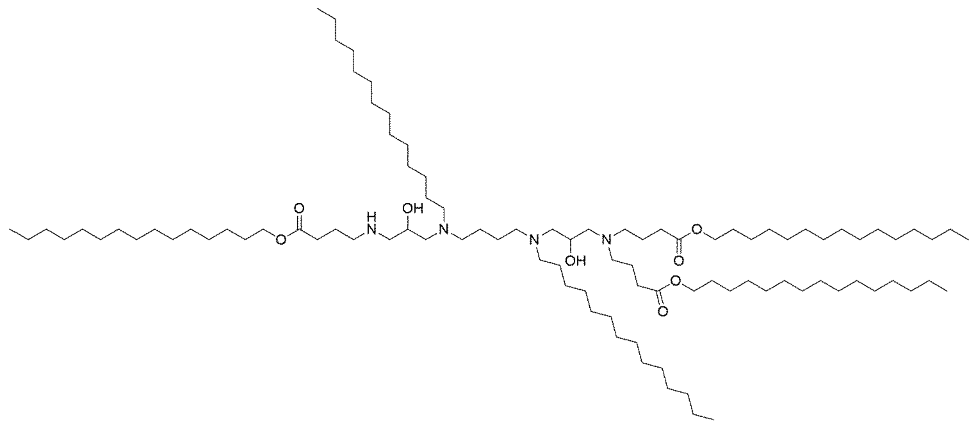 Formula (215);

The helper lipid is a cholesterol, a cholesterol analogue and/or a cholesterol derivative.

The pegylated lipid is 1,2-dipalmitoyl-sn-glycero-3-phosphatidylethanolamine-N-[methoxy(polyethylene glycol)]-2000.

In some embodiments, the molar ratio among the organic amine, the helper lipid, and the pegylated lipid in the pharmaceutical composition is (19.7-80):(19.7-80):(0.3-50); for example, the molar ratio may be (50-70):(20-40):(3-20).

In some embodiments, the pharmaceutical composition particles formed by the siRNA of the present disclosure

and the above amine-containing transfection agent have an average diameter from about 30 nm to about 200 nm, typically from about 40 nm to about 135 nm, and more typically, the average diameter of the liposome particles is from about 50 nm to about 120 nm, from about 50 nm to about 100 nm, from about 60 nm to about 90 nm, or from about 70 nm to about 90 nm, for example, the average diameter of the liposome particles is about 30, 40, 50, 60, 70, 75, 80, 85, 90, 100, 110, 120, 130, 140, 150 or 160 nm.

In some embodiments, in the pharmaceutical composition formed by the siRNA of the present disclosure and the above amine-containing transfection agent, the weight ratio (weight/weight ratio) of the siRNA to total lipids (e.g., the organic amine, the helper lipid and/or the pegylated lipid), ranges from about 1:1 to about 1:50, from about 1:1 to about 1:30, from about 1:3 to about 1:20, from about 1:4 to about 1:18, from about 1:5 to about 1:17, from about 1:5 to about 1:15, from about 1:5 to about 1:12, from about 1:6 to about 1:12, or from about 1:6 to about 1:10. For example, the weight ratio of the siRNA of the present disclosure to the total lipids is about 1:5, 1:6, 1:7, 1:8, 1:9, 1:10, 1:11, 1:12, 1:13, 1:14, 1:15, 1:16, 1:17 or 1:18.

In some embodiments, the pharmaceutical composition may be marketed with each component being separate, and used in the form of a liquid formulation. In some embodiments, the pharmaceutical composition formed by the siRNA of the present disclosure and the above pharmaceutically acceptable carrier may be prepared by various known processes, except replacing the existing siRNA with the siRNA of the present disclosure. In some embodiments, the pharmaceutical composition may be prepared according to the following process.

The organic amine, the helper lipid and the pegylated lipid are suspended in alcohol at a molar ratio as described above and mixed homogeneously to yield a lipid solution; and the alcohol is used in an amount such that the resultant lipid solution is present at a total mass concentration of 2 to 25 mg/mL, e.g., 8 to 18 mg/mL. The alcohol is selected from pharmaceutically acceptable alcohols, such as an alcohol that is in liquid form near room temperature, for example, one or more of ethanol, propylene glycol, benzyl alcohol, glycerol, PEG 200, PEG 300, PEG 400, and for example, may be ethanol.

The siRNA provided by the present disclosure is dissolved in a buffered salt solution to obtain an aqueous solution of the siRNA. The buffered salt solution has a concentration of 0.05-0.5 M, e.g., may be 0.1-0.2 M. The pH of the buffered salt solution is adjusted to 4.0-5.5, e.g., may be 5.0-5.2. The buffered salt solution is used in an amount such that the siRNA is present at a concentration of no more than 0.6 mg/ml, e.g., may be 0.2-0.4 mg/mL. The buffered salt may be one or more selected from the group consisting of soluble acetate and soluble citrate, e.g., may be sodium acetate and/or potassium acetate.

The lipid solution and the aqueous solution of the siRNA are mixed. The product obtained after mixing is incubated at a temperature of 40-60°C for at least 2 minutes (e.g., may be 5-30 minutes) to produce an incubated liposome formulation. The volume ratio of the lipid solution to the aqueous solution of the siRNA is 1:(2-5), for example, may be 1:4.

The incubated liposome formulation is concentrated or diluted, purified to remove impurities, and then sterilized to obtain the pharmaceutical composition provided by the present disclosure, which has physicochemical parameters as follows: a pH of 6.5-8, an encapsulation percentage of not less than 80%, a particle size of 40-200 nm, a polydispersity index of not higher than 0.30, and an osmotic pressure of 250-400 mOsm/kg; for example, the physicochemical parameters may be as follows: a pH of 7.2-7.6, an encapsulation percentage of not less than 90%, a particle size of 60-100 nm, a polydispersity index of not higher than 0.20, and an osmotic pressure of 300-400 mOsm/kg.

The concentration or dilution step may be performed before, after or simultaneously with the step of impurity removal. The method for removing impurities may be any of various existing methods, for example, ultrafiltration using tangential flow system viahollow fiber column at 100 KDa and with a phosphate buffer solution (PBS) at pH 7.4 as an ultrafiltration exchange solution. The method for sterilization may be any of various existing methods, such as filtration sterilization on a 0.22 μm filter.

<u>siRNA conjugate</u>

In yet another aspect, the present disclosure provides an siRNA conjugate, wherein the siRNA conjugate comprises the above-mentioned siRNA and a conjugating group conjugated to the siRNA.

Generally, the conjugating group comprises at least one pharmaceutically acceptable targeting group and an optional linker. Moreover, the siRNA, the linker and the targeting group are linked in succession. In some embodiments, there are 1-6 targeting groups. In some embodiments, there are 2-4 targeting groups. The siRNA molecule may be non-covalently or covalently conjugated to the conjugating group, for example, the siRNA molecule may be covalently conjugated to the conjugating group. The conjugating site between the siRNA and the conjugating group may be at 3'-terminal or 5'-terminal of the sense strand of the siRNA, or at 5'-terminal of the antisense strand, or may be within the internal sequence of the siRNA. In some embodiments, the conjugating site between the siRNA and the conjugating group is at 3' terminal of the sense strand of the siRNA.

In some embodiments, the conjugating group may be linked to a phosphate group, the 2'-hydroxy or the base

of a nucleotide. In some embodiments, the conjugating group may be linked to a 3'-hydroxy when the nucleotides are linked to each other via a 2'-5'-phosphodiester bond. When the conjugating group is linked to a terminal of the siRNA, the conjugating group is typically linked to the phosphate group of a nucleotide; when the conjugating group is linked to the internal sequence of the siRNA, the conjugating group is typically linked to a ribose ring or a base. For various linking modes, reference may be made to: Muthiah Manoharan et.al, siRNA conjugates carrying sequentially assembled trivalent N-acetylgalactosamine linked through nucleosides elicit robust gene silencing in vivo in hepatocytes. ACS Chemical biology, 2015,10(5):1181-1187.

In some embodiments, the siRNA and the conjugating group may be linked by an acid labile or reducible chemical bond, and these chemical bonds may be degraded under the acidic environment of cell endosomes, thereby rendering the siRNA to be in free state. For non-degradable conjugating modes, the conjugating group may be linked to the sense strand of the siRNA, thereby minimizing the effect of conjugation on the activity of the siRNA.

In some embodiments, the pharmaceutically acceptable targeting group may be a conventionally used ligand in the field of siRNA administration, for example, the various ligands as described in WO2009082607A2, which is incorporated herein by reference in its entirety.

In some embodiments, the pharmaceutically acceptable targeting group may be selected from one or more of the ligands formed by the following targeting molecules or derivatives thereof: lipophilic molecules, such as cholesterol, bile acids, vitamins (such as vitamin E), lipid molecules of different chain lengths; polymers, such as polyethylene glycol; polypeptides, such as cell-penetrating peptide; aptamers; antibodies; quantum dots; saccharides, such as lactose, polylactose, mannose, galactose, and N-acetylgalactosamine (GalNAc); folate; and receptor ligands expressed in hepatic parenchymal cells, such as asialoglycoprotein, asialo-sugar residue, lipoproteins (such as high density lipoprotein, low density lipoprotein), glucagon, neurotransmitters (such as adrenaline), growth factors, transferrin and the like.

In some embodiments, each ligand is independently selected from a ligand capable of binding to a cell surface receptor. In some embodiments, at least one ligand is a ligand capable of binding to a hepatocyte surface receptor. In some embodiments, at least one ligand is a ligand capable of binding to a mammalian cell surface receptor. In some embodiments, at least one ligand is a ligand capable of binding to a human cell surface receptor. In some embodiments, at least one ligand is a ligand capable of binding to a hepatic surface asialoglycoprotein receptor (ASGP-R). The types of these ligands are well-known to those skilled in the art and they typically serve the function of binding to specific receptors on the surface of the target cell, thereby mediating delivery of the siRNA linked to the ligand into the target cell.

In some embodiments, the pharmaceutically acceptable targeting group may be any ligand that binds to asialoglycoprotein receptors (ASGP-R) on the surface of mammalian hepatocytes. In one embodiment, each ligand is independently an asialoglycoprotein, such as asialoorosomucoid (ASOR) or asialofetuin (ASF). In some embodiments, the ligand is a saccharide or a saccharide derivative.

In some embodiments, at least one ligand is a saccharide. In some embodiments, each ligand is a saccharide. In some embodiments, at least one ligand is a monosaccharide, polysaccharide, modified monosaccharide, modified polysaccharide, or saccharide derivative. In some embodiments, at least one ligand may be a monosaccharide, disaccharide or trisaccharide. In some embodiments, at least one ligand is a modified saccharide. In some embodiments, each ligand is a modified saccharide. In some embodiments, each ligand is independently selected from the group consisting of polysaccharides, modified polysaccharides, monosaccharides, modified monosaccharides, polysaccharide derivatives or monosaccharide derivatives. In some embodiments, each ligand or at least one ligand is selected from the group consisting of the following saccharides: glucose and derivative thereof, mannose and derivative thereof, galactose and derivative thereof, xylose and derivative thereof, ribose and derivative thereof, fucose and derivative thereof, lactose and derivative thereof, maltose and derivative thereof, arabinose and derivative thereof, fructose and derivative thereof, and sialic acid.

In some embodiments, each ligand may be independently selected from D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-α-neuraminic acid, 5-thio-β-D-glucofuranose, methyl 2,3,4-tris-O-acetyl-1-thio-6-O-trityl-α-D-glucopyranoside, 4-thio-β-D-galactopyranose, ethyl 3,4,6,7-tetra-O-acetyl-2-deoxy-1, 5-dithio-a-D-glucoheptopyranoside, 2,5-anhydro-D-allononitrile, ribose, D-ribose, D-4-thioribose, L-ribose, or L-4-thioribose. Other selections for the ligand may be found, for example, in the description of CN105378082A, which is incorporated herein by reference in its entirety.

In some embodiments, the pharmaceutically acceptable targeting group in the siRNA conjugate may be galactose or N-acetylgalactosamine, wherein the galactose or N-acetylgalactosamine molecules may be monovalent, bivalent, trivalent and tetravalent. It should be understood that the terms monovalent, bivalent, trivalent and tetravalent

described herein respectively mean that the molar ratio of the siRNA molecule to the galactose or N-acetylgalactosamine molecule in the siRNA conjugate is 1:1, 1:2, 1:3 or 1:4, when the siRNA conjugate is formed by the siRNA molecule and the conjugating group containing galactose or N-acetylgalactosamine as the targeting group. In some embodiments, the pharmaceutically acceptable targeting group is N-acetylgalactosamine. In some embodiments, when the siRNA of the present disclosure is conjugated to a conjugating group comprising N-acetylgalactosamine, the N-acetylgalactosamine molecule is trivalent or tetravalent. In some embodiments, when the siRNA of the present disclosure is conjugated to a conjugating group containing N-acetylgalactosamine, the N-acetylgalactosamine molecule is trivalent.

The targeting group may be linked to the siRNA molecule via an appropriate linker, and the appropriate linker may be selected by those skilled in the art according to the specific type of the targeting group. The types of these linkers and targeting groups and the linking modes to the siRNA may be found in the disclosure of WO2015006740A2, which is incorporated herein by reference in its entirety.

In some embodiments, a linker in the present disclosure may be a structure as shown by Formula (301):

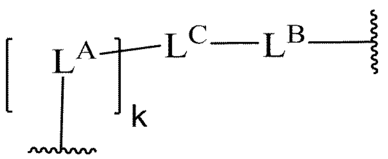

Formula (301),

wherein,

k is an integer of 1-3; and

$L^A$ has an amide bond-comprising structure as shown by Formula (302), $L^B$ has an N-acylpyrrolidine-comprising structure as shown by Formula (303) with carbonyl group and oxygen atom, and $L^C$ is a linking group based on hydroxymethyl aminomethane, dihydroxymethyl aminomethane or trihydroxymethyl aminomethane;

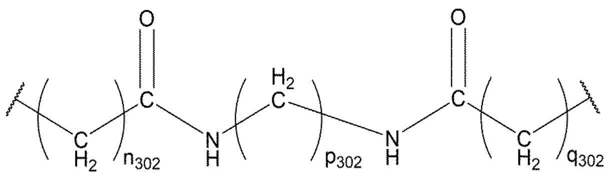

Formula (302);

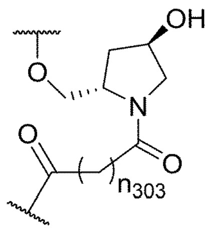

Formula (303);

wherein $n_{302}$, $q_{302}$ and $p_{302}$ are each independently an integer of 2-6, optionally, $n_{302}$, $q_{302}$ and $p_{302}$ are each independently 2 or 3; $n_{303}$ is an integer of 4-16, and optionally, $n_{303}$ is an integer of 8-12,  indicating a site of covalent connection of groups.

In the linker, each $L^A$ is respectively linked to one targeting group through an ether bond and is connected with the $L^C$ part by an ether bond formed between an oxygen atom of hydroxyl in the $L^C$ part; $L^B$ is connected by amide bond between carbonyl group in formula (303) and nitrogen atom of amino group in $L^C$ part, and is connected by a phosphoester bond or a phosphorothioate bond formed between an oxygen atom in formula (303) and the siRNA.

In some embodiments, the siRNA conjugate provided by the present disclosure has a structure as shown by Formula (305):

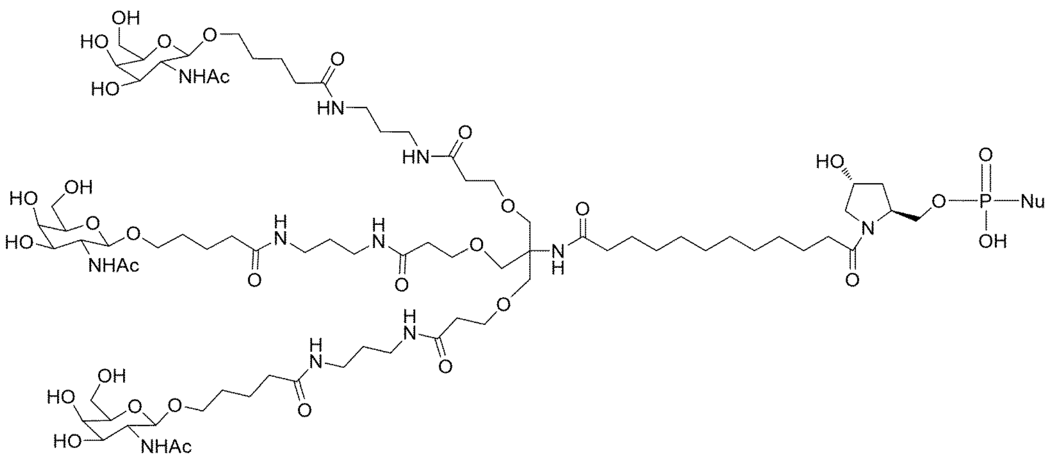

Formula (305),

wherein Nu represents the siRNA provided by the present disclosure.

In some embodiments, an linker in the siRNA conjugate of the present disclosure has a structure as shown by Formula (306):

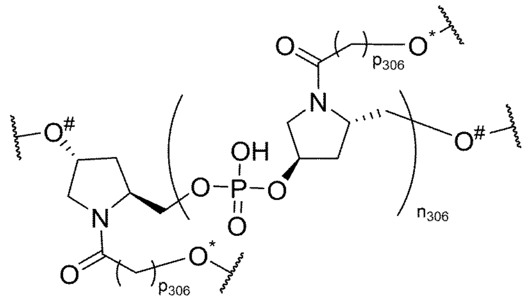

Formula (306),

wherein, $n_{306}$ is an integer of 0-3, each $p_{306}$ is independently an integer of 1-6,  represents a site where the group is covalently linked; the linking group forms ether bond connection with the targeting group through the oxygen atoms marked by *; at least one of the oxygen atoms marked by # forms a phosphoester bond or a phosphorothioate bond with the siRNA, and the rest of the oxygen atoms marked by # form a hydroxyl group or a $C_1$-$C_3$ alkyl to form a $C_1$-$C_3$ alkoxy.

In some embodiments, the siRNA conjugate of the present disclosure has a structure as shown by Formula (307):

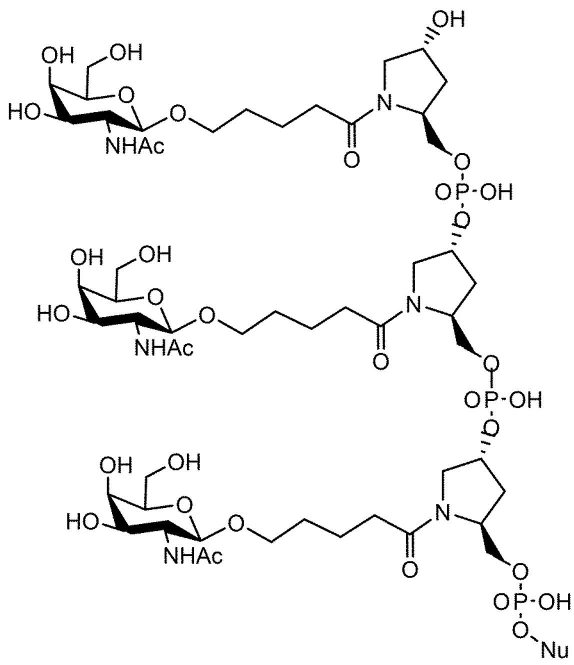

Formula (307),

wherein Nu represents the siRNA provided by the present disclosure.

In some embodiments, the siRNA conjugate has a structure as shown by Formula (308):

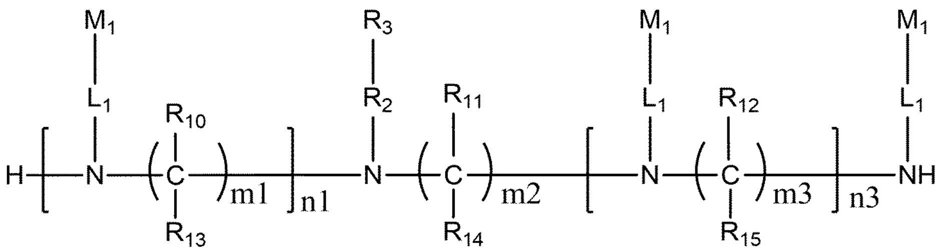

Formula (308),

wherein:

n1 is an integer selected from 1-3, and n3 is an integer selected from 0-4;

m1, m2, or m3 is independently an integer selected from 2-10;

$R_{10}$, $R_{11}$, $R_{12}$, $R_{13}$, $R_{14}$ or $R_{15}$ is each independently H or selected from the group consisting of $C_1$-$C_{10}$ alkyl, $C_1$-$C_{10}$ haloalkyl and $C_1$-$C_{10}$ alkoxy; and

$R_3$ is a group having a structure as shown by Formula A59:

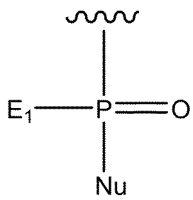

(A59)

wherein, $E_1$ is OH, SH or $BH_2$, and Nu is the siRNA of the present disclosure;

$R_2$ is a linear alkylene of 1-20 carbon atoms in length, wherein one or more carbon atoms are optionally replaced

with any one or more of the group consisting of: C(O), NH, O, S, CH=N, $S(O)_2$, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $R_2$ may optionally have any one or more substituents in the group consisting of: $C_1$-$C_{10}$ alkyl, $C_6$-$C_{10}$ aryl, $C_5$-$C_{10}$ heteroaryl, $C_1$-$C_{10}$ haloalkyl, $-OC_1$-$C_{10}$ alkyl, $-OC_1$-$C_{10}$ alkylphenyl, $-C_1$-$C_{10}$ alkyl-OH, $-OC_1$-$C_{10}$ haloalkyl, $-SC_1$-$C_{10}$ alkyl, $-SC_1$-$C_{10}$ alkylphenyl, $-C_1$-$C_{10}$ alkyl-SH, $-SC_1$-$C_{10}$ haloalkyl, halo substituent, -OH, -SH, $-NH_2$, $-C_1$-$C_{10}$ alkyl-$NH_2$, $-N(C_1$-$C_{10}$ alkyl)($C_1$-$C_{10}$ alkyl), $-NH(C_1$-$C_{10}$ alkyl), $-N(C_1$-$C_{10}$ alkyl)($C_1$-$C_{10}$ alkylphenyl), $-NH(C_1$-$C_{10}$ alkylphenyl), cyano, nitro, $-CO_2H$, $-C(O)O(C_1$-$C_{10}$ alkyl), $-CON(C_1$-$C_{10}$ alkyl)($C_1$-$C_{10}$ alkyl), $-CONH(C_1$-$C_{10}$ alkyl), $-N(C_1$-$C_{10}$ alkyl)($C_1$-$C_{10}$ alkylphenyl), $-NH(C_1$-$C_{10}$ alkylphenyl), $-CONH_2$, $-NHC(O)(C_1$-$C_{10}$ alkyl), -NHC(O)(phenyl), $-N(C_1$-$C_{10}$ alkyl)C(O)($C_1$-$C_{10}$ alkyl), $-N(C_1$-$C_{10}$ alkyl)C(O)(phenyl), $-C(O)C_1$-$C_{10}$ alkyl, $-C(O)C_1$-$C_{10}$ alkylphenyl, $-C(O)C_1$-$C_{10}$ haloalkyl, $-OC(O)C_1$-$C_{10}$ alkyl, $-SO_2(C_1$-$C_{10}$ alkyl), $-SO_2$(phenyl), $-SO_2(C_1$-$C_{10}$ haloalkyl), $-SO_2NH_2$, $-SO_2NH(C_1$-$C_{10}$ alkyl), $-SO_2NH$(phenyl), $-NHSO_2(C_1$-$C_{10}$ alkyl), $-NHSO_2$(phenyl), and $-NHSO_2(C_1$-$C_{10}$ haloalkyl); and

each $L_1$ is a linear alkylene of 1-70 carbon atoms in length, wherein one or more carbon atoms are optionally replaced with any one or more of the group consisting of: C(O), NH, O, S, CH=N, $S(O)_2$, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $L_1$ may optionally have any one or more substituents in the group consisting of: $C_1$-$C_{10}$ alkyl, $C_6$-$C_{10}$ aryl, $C_5$-$C_{10}$ heteroaryl, $C_1$-$C_{10}$ haloalkyl, $-OC_1$-$C_{10}$ alkyl, $-OC_1$-$C_{10}$ alkylphenyl, $-C_1$-$C_{10}$ alkyl-OH, $-OC_1$-$C_{10}$ haloalkyl, $-SC_1$-$C_{10}$ alkyl, $-SC_1$-$C_{10}$ alkylphenyl, $-C_1$-$C_{10}$ alkyl-SH, $-SC_1$-$C_{10}$ haloalkyl, halo substituent, -OH, -SH, $-NH_2$, $-C_1$-$C_{10}$ alkyl-$NH_2$, $-N(C_1$-$C_{10}$ alkyl)($C_1$-$C_{10}$ alkyl), $-NH(C_1$-$C_{10}$ alkyl), $-N(C_1$-$C_{10}$ alkyl)($C_1$-$C_{10}$ alkylphenyl), $-NH(C_1$-$C_{10}$ alkylphenyl), cyano, nitro, $-CO_2H$, $-C(O)O(C_1$-$C_{10}$ alkyl), $-CON(C_1$-$C_{10}$ alkyl)($C_1$-$C_{10}$ alkyl), $-CONH(C_1$-$C_{10}$ alkyl), $-N(C_1$-$C_{10}$ alkyl)($C_1$-$C_{10}$ alkylphenyl), $-NH(C_1$-$C_{10}$ alkylphenyl), $-CONH_2$, $-NHC(O)(C_1$-$C_{10}$ alkyl), -NHC(O)(phenyl), $-N(C_1$-$C_{10}$ alkyl)C(O)($C_1$-$C_{10}$ alkyl), $-N(C_1$-$C_{10}$ alkyl)C(O)(phenyl), $-C(O)C_1$-$C_{10}$ alkyl, $-C(O)C_1$-$C_{10}$ alkylphenyl, $-C(O)C_1$-$C_{10}$ haloalkyl, $-OC(O)C_1$-$C_{10}$ alkyl, $-SO_2(C_1$-$C_{10}$ alkyl), $-SO_2$(phenyl), $-SO_2(C_1$-$C_{10}$ haloalkyl), $-SO_2NH_2$, $-SO_2NH(C_1$-$C_{10}$ alkyl), $-SO_2NH$(phenyl), $-NHSO_2(C_1$-$C_{10}$ alkyl), $-NHSO_2$(phenyl), and $-NHSO_2(C_1$-$C_{10}$ haloalkyl).

In some embodiments, $L_1$ may be selected from the group consisting of groups A1-A26 and any combination thereof, wherein the structures and definitions of A1-A26 are as follows:

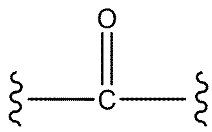,
(A1)

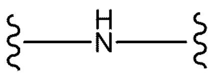,
(A2)

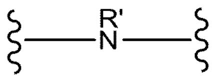,
(A3)

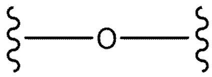,
(A4)

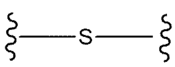,
(A5)

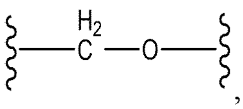,
(A6)

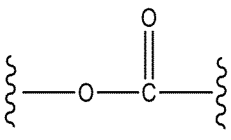,
(A7)

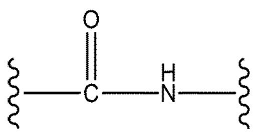,
(A8)

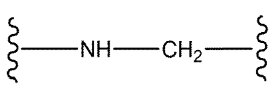,
(A9)

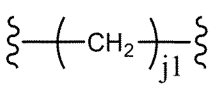,
(A10)

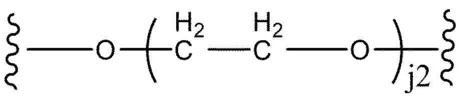,
(A11)

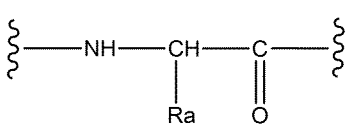,

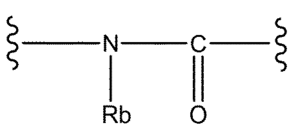,

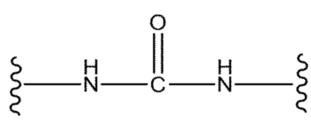,

(A12) (A13) (A14)

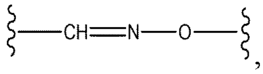, 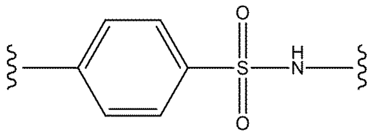, 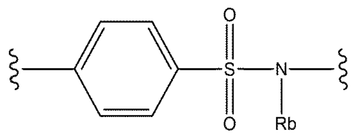,

(A15) (A16) (A17)

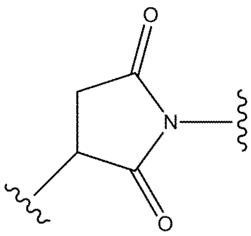, 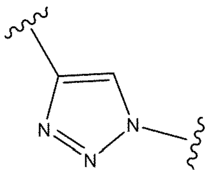, 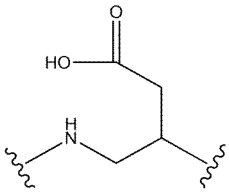, 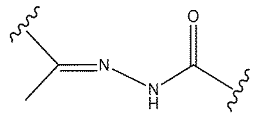,

(A18) (A19) (A20) (A21)

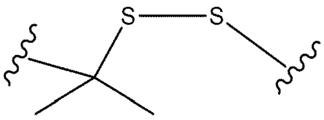, 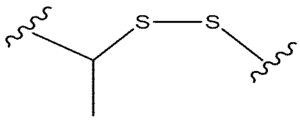, 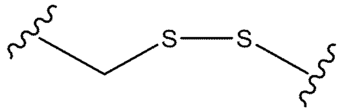,

(A22) (A23) (A24)

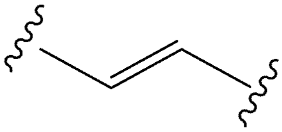, 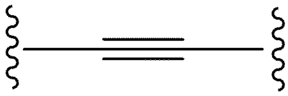;

(A25) (A26)

wherein, j1 is each independently an integer of 1-20; and j2 is each independently an integer of 1-20;
R' is a $C_1$-$C_{10}$ alkyl; and
Ra is selected from the group consisting of groups A27-A45 and any combinations thereof:

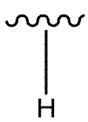, 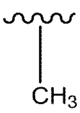, 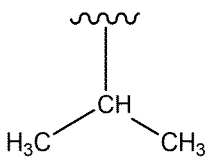, 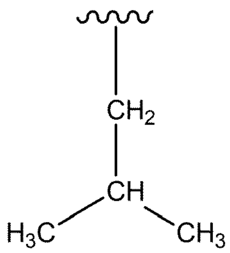, 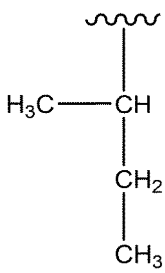, 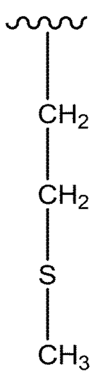,

(A27) (A28) (A29) (A30) (A31) (A32)

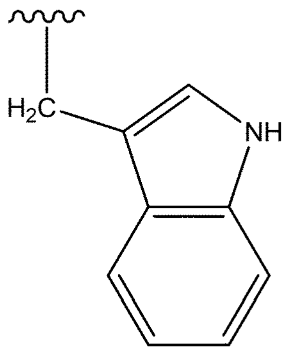, 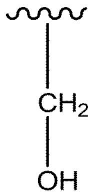, 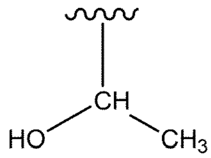, 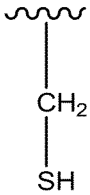, 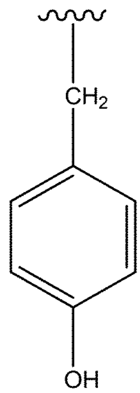,

(A33) (A34) (A35) (A36) (A37)

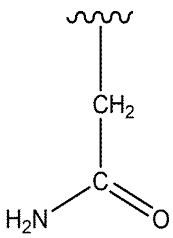, 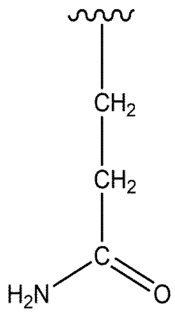, 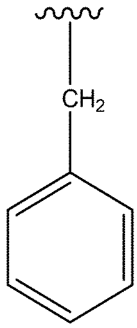, 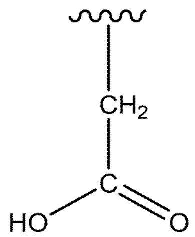, 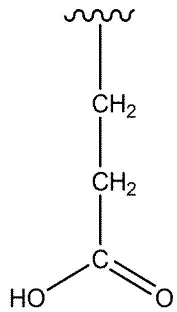,

(A38) (A39) (A40) (A41) (A42)

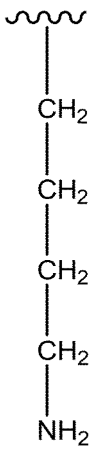, 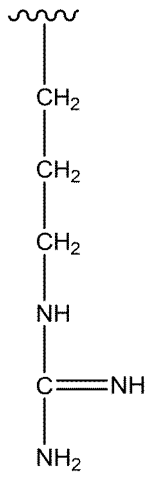 or 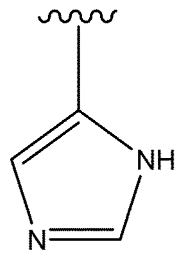;

(A43) (A44) (A45)

Rb is a $C_1$-$C_{10}$ alkyl; and

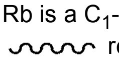 represents a site where the group is covalently linked.

Those skilled in the art would understand that, though $L_1$ is defined as a linear alkylene for convenience, but it may not be a linear group or be named differently, such as an amine or alkenyl produced by the above replacement and/or substitution. For the purpose of the present disclosure, the length of $L_1$ is the number of the atoms in the chain connecting the two attachment points. For this purpose, a ring obtained by replacement of a carbon atom of the linear alkylene, such as a heterocyclylene or heteroarylene, is counted as one atom.

$M_1$ represents a targeting group, of which the definitions and options are the same as those described above. In some embodiments, each $M_1$ is independently selected from one of the ligands that have affinity to the asialoglycoprotein receptor on the surface of mammalian hepatocytes.

When $M_1$ is a ligand that has affinity to the asialoglycoprotein receptor on the surface of mammalian hepatocytes, in some embodiments, nl may be an integer of 1-3, and n3 may be an integer of 0-4 to ensure that the number of the $M_1$ targeting group in the siRNA conjugate may be at least 2. In some embodiments, n1+n3≥2, such that the number of the $M_1$ targeting group in the conjugate may be at least 3, thereby allowing the $M_1$ targeting group to more easily bind to the asialoglycoprotein receptor on the surface of hepatocytes, which may facilitate the endocytosis of the siRNA conjugate into cells. Experiments have shown that when the number of the $M_1$ targeting group is greater than 3, the ease of binding the $M_1$ targeting group to the asialoglycoprotein receptor on the surface of hepatocytes is not significantly increased. Therefore, in view of various aspects such as synthesis convenience, structure/process costs and delivery efficiency, in some embodiments, n1 is an integer of 1-2, n3 is an integer of 0-1, and n1+n3=2-3.

In some embodiments, when ml, m2, or m3 is independently selected from an integer of 2-10, the steric mutual positions among a plurality of $M_1$ targeting groups may be fit for the binding of the $M_1$ targeting groups to the asialoglycoprotein receptor on the surface of hepatocytes. In order to make the siRNA conjugate provided by the present disclosure simpler, easier to synthesis and/or reduce cost, in some embodiments, ml, m2 and m3 are each independently an integer of 2-5, and in some embodiments, ml=m2=m3.

Those skilled in the art would understand that when $R_{10}$, $R_{11}$, $R_{12}$, $R_{13}$, $R_{14}$, or $R_{15}$ is each independently selected from one of H, $C_1$-$C_{10}$ alkyl, $C_1$-$C_{10}$ haloalkyl, and $C_1$-$C_{10}$ alkoxy, they would not change the properties of the siRNA conjugate of the present disclosure and all could achieve the purpose of the present disclosure. In some embodiments, $R_{10}$, $R_{11}$, $R_{12}$, $R_{13}$, $R_{14}$, or $R_{15}$ is each independently selected from H, methyl or ethyl. In some embodiments, $R_{10}$, $R_{11}$, $R_{12}$, $R_{13}$, $R_{14}$, and $R_{15}$ are all H.

$R_3$ is a group having the structure as shown by Formula A59, wherein $E_1$ is OH, SH or $BH_2$, and considering the availability of starting materials, in some embodiments, $E_1$ is OH or SH.

$R_2$ is selected to achieve the linkage between the group as shown by Formula A59 and the N atom on a nitrogenous backbone. In the context of the present disclosure, the "nitrogenous backbone" refers to a chain structure in which the carbon atoms attached to $R_{10}$, Rn, $R_{12}$, $R_{13}$, $R_{14}$, and $R_{15}$ and the N atoms are linked to each other. Therefore, $R_2$ may be any linking group capable of attaching the group A59 to the N atom on a nitrogenous backbone by suitable means. In some embodiments, in the case where the siRNA conjugate as shown by Formula (308) is prepared by a solid phase synthesis process, $R_2$ group needs to have both a site linking to the N atom on the nitrogenous backbone and a site linking to the P atom in $R_3$. In some embodiments, in $R_2$, the site linking to the N atom on the nitrogenous backbone forms an amide bond with the N atom, and the site linking to the P atom in $R_3$ forms a phosphoester bond with the P atom. In some embodiments, $R_2$ may be B5, B6, B5' or B6':

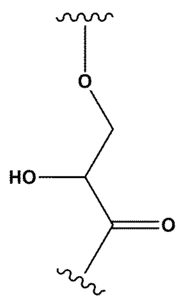
,
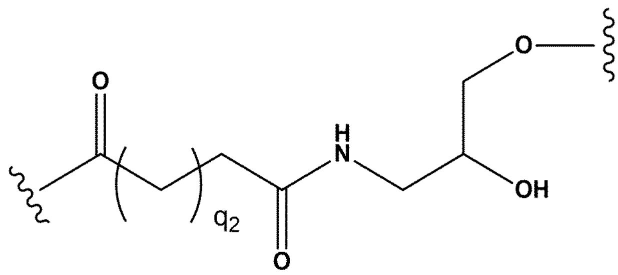
;

(B5) (B6),

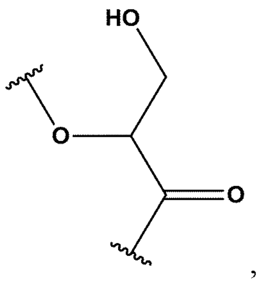
,
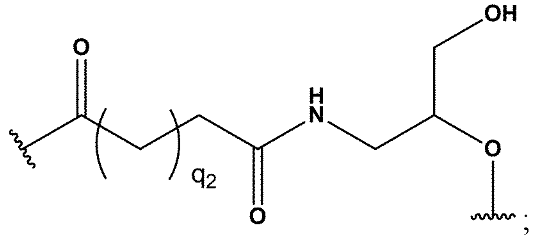
;

(B5') (B6'),

wherein,  represents a site where the group is covalently linked.

A value range of $q_2$ may be an integer of 1-10; and in some embodiments, $q_2$ is an integer of 1-5.

The role of $L_1$ is to link the $M_1$ targeting group to the N atom on the nitrogenous backbone, thereby providing liver targeting function for the siRNA conjugate as shown by Formula (308). In some embodiments, $L_1$ is selected from the connection combinations of one or more of groups Al-A26. In some embodiments, $L_1$ is selected from the connection combinations of one or more of A1, A4, A5, A6, A8, A10, A11, and A13. In some embodiments, $L_1$ is selected from the connection combinations of at least two of A1, A4, A8, A10, and A11. In some embodiments, $L_1$ is selected from the connection combinations of at least two of A1, A8, and A10.

In some embodiments, the length of $L_1$ may be 3-25 atoms, 3-20 atoms, 4-15 atoms or 5-12 atoms. In some embodiments, the length of $L_1$ is 3, 4, 5, 6, 7, 8, 9, 10, 11, 12, 13, 14, 15, 16, 17, 18, 19, 20, 21, 22, 23, 24, 25, 30, 35, 40, 45, 50, 55, 60 atoms.

In some embodiments, each j 1 is independently an integer of 2-10, and in some embodiments, j 1 is an integer of 3-5. In some embodiments, each j2 is independently an integer of 2-10, and in some embodiments, each j2 is an integer of 3-5. R' is a $C_1$-$C_4$ alkyl, and in some embodiments, R' is one of methyl, ethyl, and isopropyl. Ra is one of A27, A28, A29, A30, and A31, and in some embodiments, Ra is A27 or A28. Rb is a $C_1$-$C_5$ alkyl, and in some embodiments, Rb is one of methyl, ethyl, isopropyl, and butyl. In some embodiments, jl, j2, R', Ra, and Rb are respectively selected by Formulae A1-A26 to achieve the linkage between the $M_1$ targeting group and the N atom on the nitrogenous backbone, and to make the steric mutual position among the $M_1$ targeting group more suitable for the binding of the $M_1$ targeting group to the asialoglycoprotein receptor on the surface of hepatocytes.

In some embodiments, the siRNA conjugate has a structure as shown by Formula (403), (404), (405), (406), (407), (408), (409), (410), (411), (412), (413), (414), (415), (416), (417), (418), (419), (420), (421) or (422):

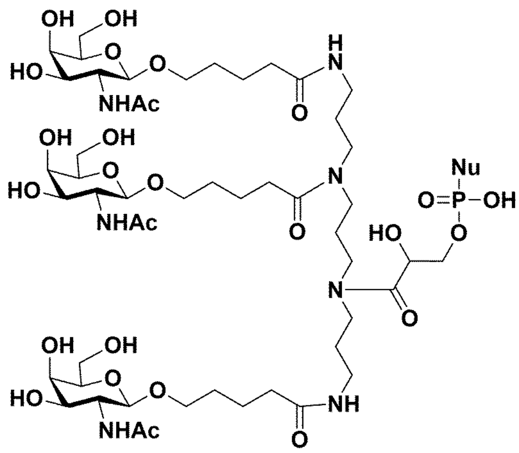

Formula (403)

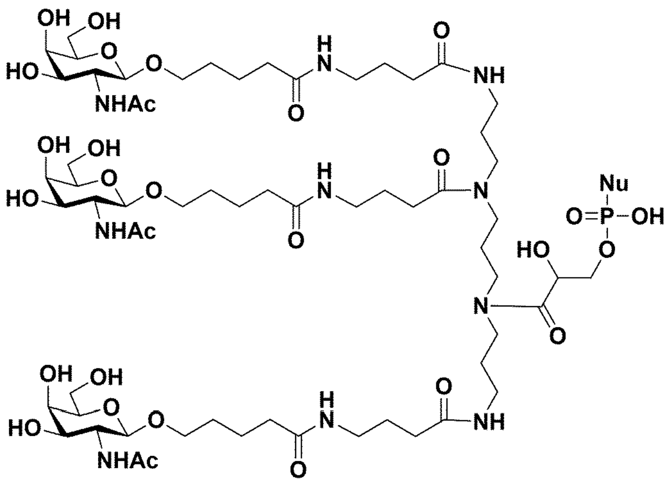

Formula (404)

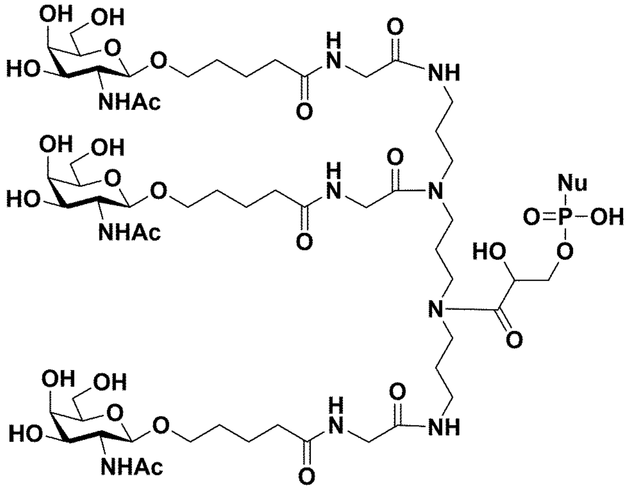

Formula (405)

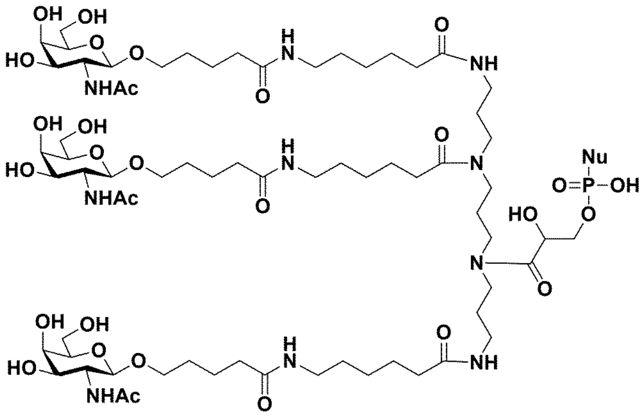

Formula (406)

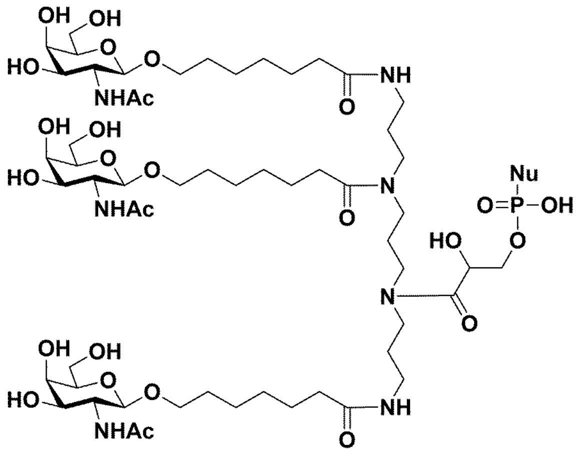

Formula (407)

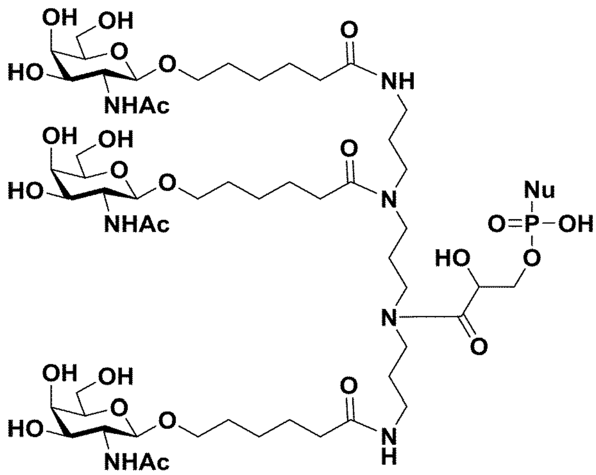

Formula (408)

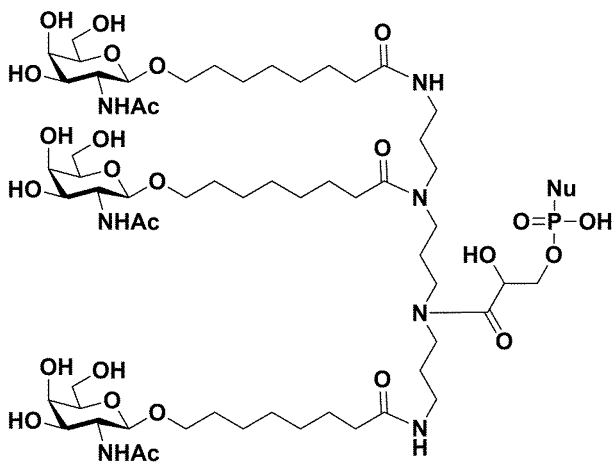

Formula (409)

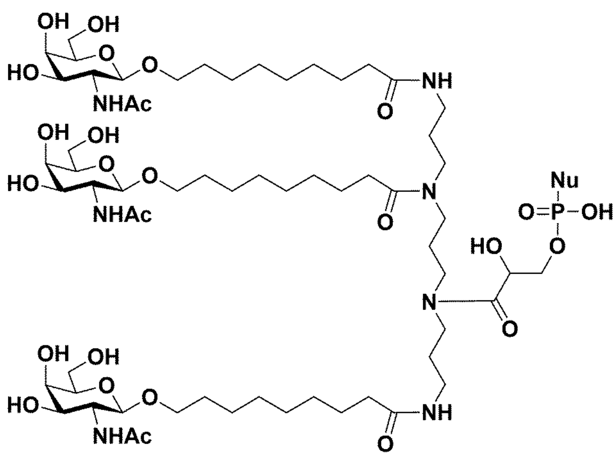

Formula (410)

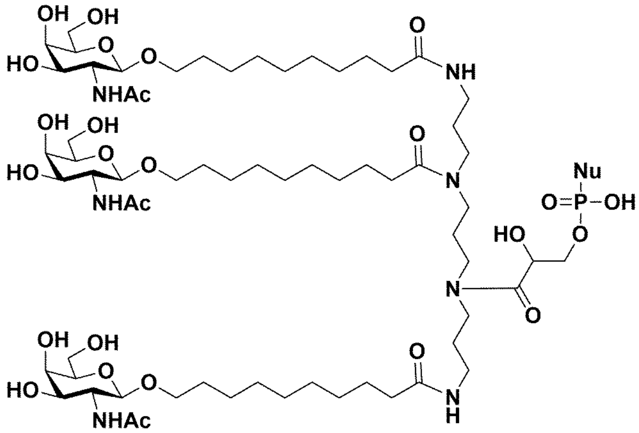

Formula (411)

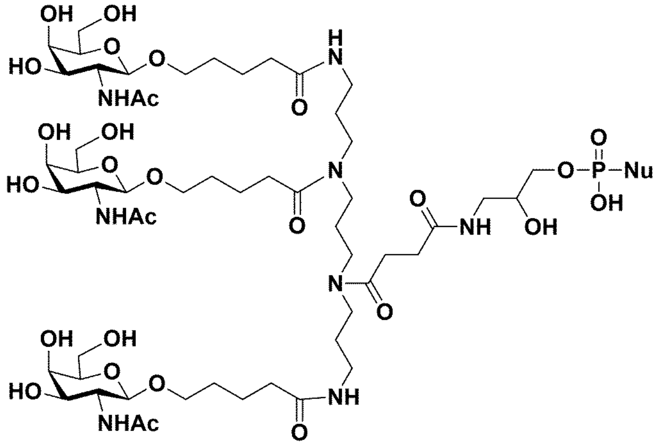

Formula (412)

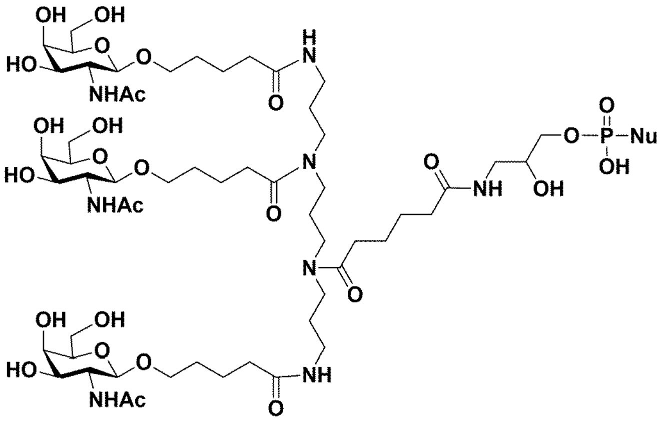

Formula (413)

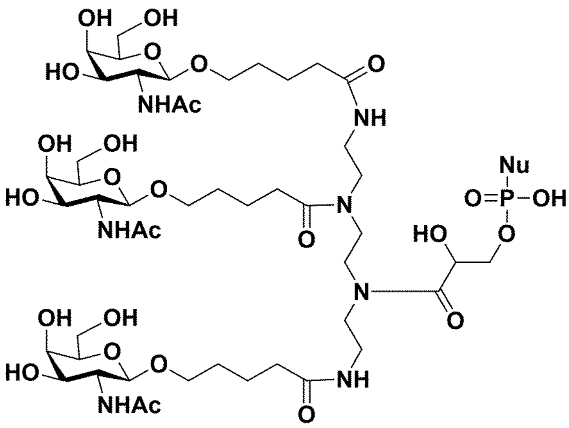

Formula (414)

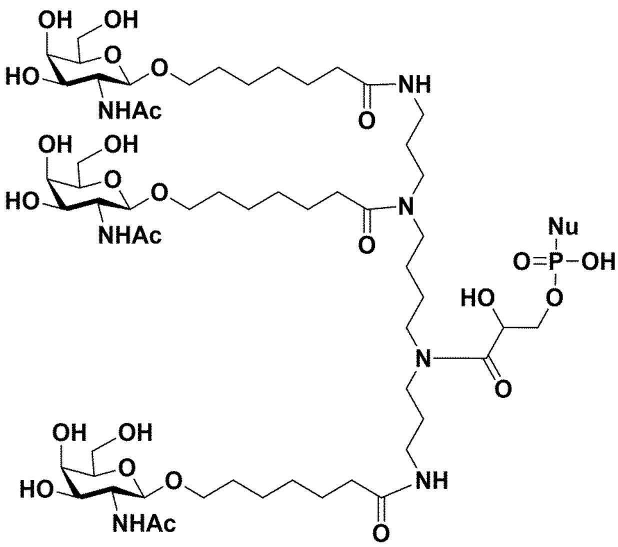

Formula (415)

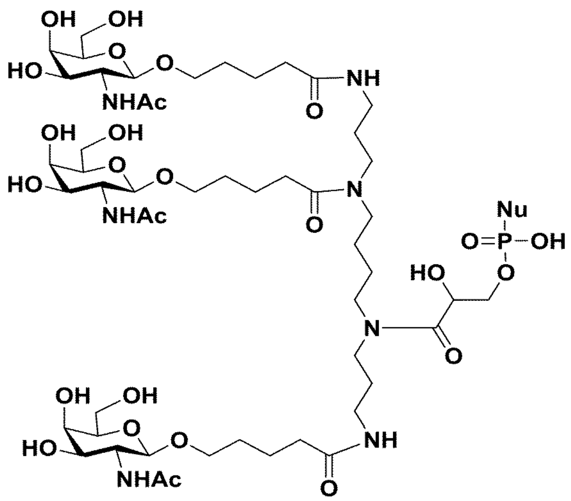

Formula (416)

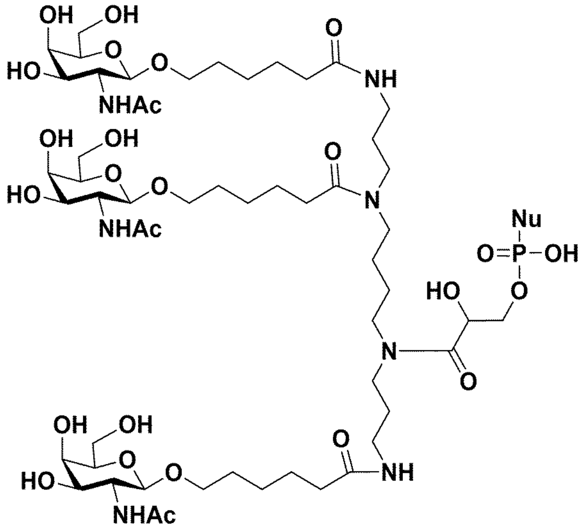

Formula (417)

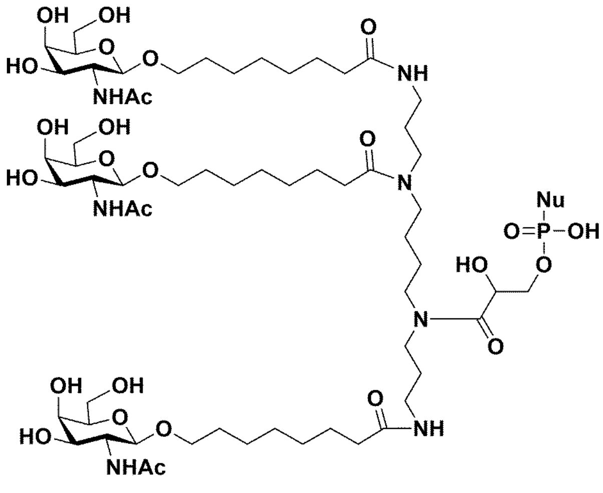

Formula (418)

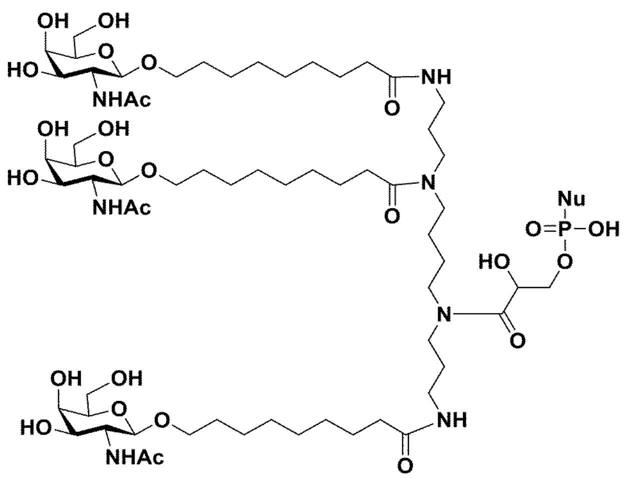

Formula (419)

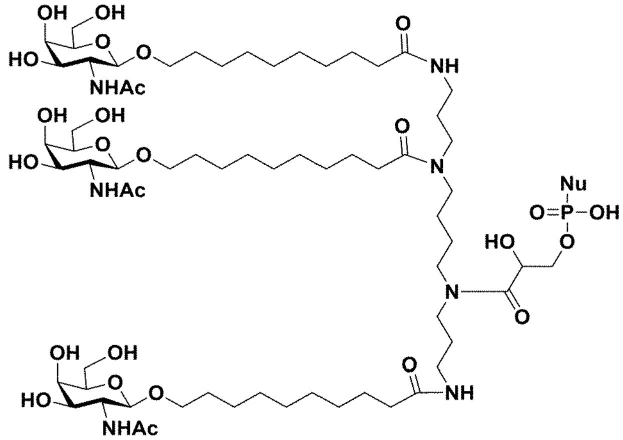

Formula (420)

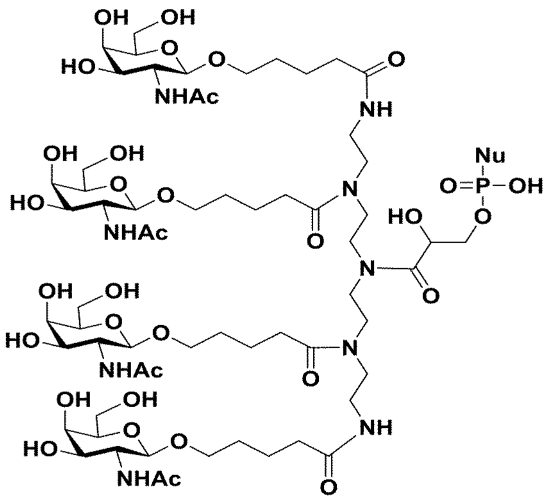

Formula (421)

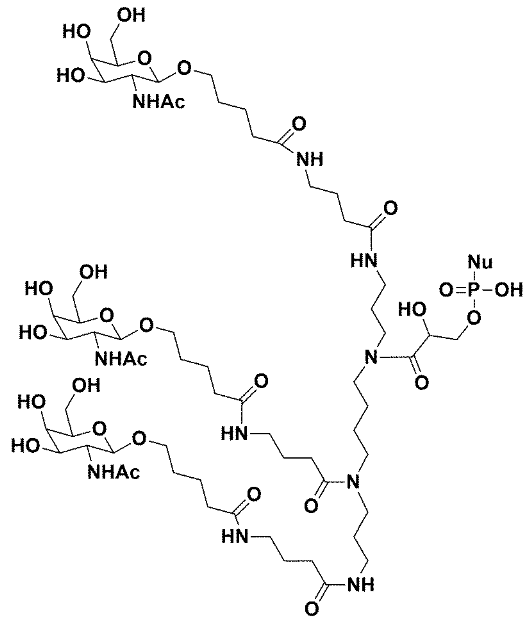

Formula (422).

In some embodiments, the P atom in Formula A59 may be linked to any possible position in the siRNA sequence, for example, the P atom in Formula A59 may be linked to any nucleotide in the sense strand or the antisense strand of the siRNA. In some embodiments, the P atom in Formula A59 is linked to any nucleotide in the sense strand of the siRNA. In some embodiments, the P atom in Formula A59 is linked to an end of the sense strand or the antisense strand of the siRNA. In some embodiments, the P atom in Formula A59 is linked to an end of the sense strand of the siRNA. The end refers to the first 4 nucleotides counted from one terminal of the sense strand or antisense strand. In some embodiments, the P atom in Formula A59 is linked to the terminal of the sense strand or the antisense strand of the siRNA. In some embodiments, the P atom in Formula A59 is linked to 3' terminal of the sense strand of the siRNA. In the case where the P atom in Formula A59 is linked to the above position in the sense strand of the siRNA, after entering into cells, the siRNA conjugate as shown by Formula (308) can release a separate antisense strand of the siRNA during unwinding, thereby blocking the translation of the PKK mRNAinto protein and inhibiting the expression of PKK gene.

In some embodiments, the P atom in Formula A59 may be linked to any possible position of a nucleotide in the siRNA, for example, to position 5', 2' or 3' of the nucleotide, or to the base of the nucleotide. In some embodiments, the P atom in Formula A59 may be linked to position 2', 3', or 5' of a nucleotide in the siRNA by forming a phosphodiester bond. In some embodiments, the P atom in Formula A59 is linked to an oxygen atom formed by deprotonation of 3'-hydroxy of the nucleotide at 3' terminal of the sense strand of the siRNA (in this case, the P atom in Formula A59 may also be regarded as a P atom in a phosphate group contained in the siRNA), or the P atom in Formula A59 is linked to a nucleotide of the sense strand of the siRNA by substituting the hydrogen in the 2'-hydroxy of the nucleotide, or the P atom in Formula A59 is linked to a nucleotide at 5' terminal of the sense strand of the siRNA by substituting the hydrogen in the 5'-hydroxy of the nucleotide.

The inventors of the present disclosure have surprisingly found that the siRNA of the present disclosure has significantly improved stability in plasma and low off-target effect, and also shows higher silencing activity against PKK mRNA. The siRNA conjugates containing these siRNAs show higher silencing activity against PKK mRNA. Therefore, in some embodiments, the siRNA of the present disclosure may be one of the siRNAs shown in Table 1.

Table 1 siRNA sequence of the present disclosure

| siRNA No. | SEQ ID NO: | Sequence direction 5'-3' |
|---|---|---|
| siPKKa1 | 9 | CUGAAUUCCAAAAACCAAU |
| | 10 | AUUGGUUUUUGGAAUUCAGUA |
| siPKKa2 | 11 | UACUGAAUUCCAAAAACCAAU |
| | 12 | AUUGGUUUUUGGAAUUCAGUAUA |
| siPKKa1-M1 | 13 | CmUmGmAmAmUmUfCfCfAmAmAmAmAmCmCmAmAmUm |
| | 14 | AmUfUmGmGmUfUmUmUmUmGmGmAmAfUmUfCmAmGmUmAm |
| siPKKa1-M2 | 15 | CmUmGmAmAfUmUfCfCfAmAmAmAmAmCmCmAmAmUm |
| | 16 | AmUfUmGmGmUfUmUfUfUmGmGmAmAfUmUfCmAmGmUmAm |
| siPKKa1-M3 | 17 | CmUmGmAmAfUmUfCfCfAmAmAmAmAmCmCmAmAmUm |
| | 18 | AmUfUmGmGmUfUmUmUmUmGmGmAmAfUmUfCmAmGmUmAm |
| siPKKa2-M1 | 19 | UmAmCmUmGmAmAmUmUfCfCfAmAmAmAmAmCmCmAmAmUm |
| | 20 | AmUfUmGmGmUfUmUmUmUmGmGmAmAfUmUfCmAmGmUmAmUmAm |
| siPKKa2-M2 | 21 | UmAmCmUmGmAmAfUmUfCfCfAmAmAmAmAmCmCmAmAmUm |
| | 22 | AmUfUmGmGmUfUmUfUfUmGmGmAmAfUmUfCmAmGmUmAmUmAm |
| siPKKa2-M3 | 23 | UmAmCmUmGmAmAfUmUfCfCfAmAmAmAmAmCmCmAmAmUm |
| | 24 | AmUfUmGmGmUfUmUmUmUmGmGmAmAfUmUfCmAmGmUmAmUmAm |
| siPKKa1-M1S | 25 | CmsUmsGmAmAmUmUfCfCfAmAmAmAmAmCmCmAmAmUm |
| | 26 | AmsUfsUmGmGmUfUmUmUmUmGmGmAmAfUmUfCmAmGmsUmsAm |
| siPKKa1-M2S | 27 | CmsUmsGmAmAfUmUfCfCfAmAmAmAmAmCmCmAmAmUm |
| | 28 | AmsUfsUmGmGmUfUmUfUfUmGmGmAmAfUmUfCmAmGmsUmsAm |

(continued)

| siRNA No. | SEQ ID NO: | Sequence direction 5'-3' |
|---|---|---|
| siPKKa1-M3S | 29 | CmsUmsGmAmAfUmUfCfCfAmAmAmAmAmCmCmAmAmUm |
| | 30 | AmsUfsUmGmGmUfUmUmUmUmGmGmAmAfUmUfCmAmGmsUmsAm |
| siPKKa2-M1S | 31 | UmsAmsCmUmGmAmAmUmUfCfCfAmAmAmAmAmCmCmAmAmUm |
| | 32 | AmsUfsUmGmGmUfUmUmUmUmGmGmAmAfUmUfCmAmGmUmAmsUmsAm |
| siPKKa2-M2S | 33 | UmsAmsCmUmGmAmAfUmUfCfCfAmAmAmAmAmCmCmAmAmUm |
| | 34 | AmsUfsUmGmGmUfUmUfUfUmGmGmAmAfUmUfCmAmGmUmAmsUmsAm |
| siPKKa2-M3S | 35 | UmsAmsCmUmGmAmAfUmUfCfCfAmAmAmAmAmCmCmAmAmUm |
| | 36 | AmsUfsUmGmGmUfUmUmUmUmGmGmAmAfUmUfCmAmGmUmAmsUmsAm |
| siPKKa1-M1P1 | 37 | CmUmGmAmAmUmUfCfCfAmAmAmAmAmCmCmAmAmUm |
| | 38 | P1AmUfUmGmGmUfUmUmUmUmGmGmAmAfUmUfCmAmGmUmAm |
| siPKKa1-M2P1 | 39 | CmUmGmAmAfUmUfCfCfAmAmAmAmAmCmCmAmAmUm |
| | 40 | P 1 AmUfUmGmGmUfUmUfUfUmGmGmAmAfUmUfCmAmGmUmAm |
| siPKKa1-M3P1 | 41 | CmUmGmAmAfUmUfCfCfAmAmAmAmAmCmCmAmAmUm |
| | 42 | P1AmUfUmGmGmUfUmUmUmUmGmGmAmAfUmUfCmAmGmUmAm |

(continued)

| siRNA No. | SEQ ID NO: | Sequence direction 5'-3' |
|---|---|---|
| siPKKa2-M1P1 | 43 | UmAmCmUmGmAmAmUmUfCfCfAmAmAmAmAmCmCmAmAmUm |
| | 44 | P1AmUfUmGmGmUfUmUmUmUmGmGmAmAfUmUfCmAmGmUmAmUmAm |
| siPKKa2-M2P1 | 45 | UmAmCmUmGmAmAfUmUfCfCfAmAmAmAmAmCmCmAmAmUm |
| | 46 | P1AmUfUmGmGmUfUmUfUfUmGmGmAmAfUmUfCmAmGmUmAmUmAm |
| siPKKa2-M3P1 | 47 | UmAmCmUmGmAmAfUmUfCfCfAmAmAmAmAmCmCmAmAmUm |
| | 48 | P1AmUfUmGmGmUfUmUmUmUmGmGmAmAfUmUfCmAmGmUmAmUmAm |
| siPKKa1-M1SP1 | 49 | CmsUmsGmAmAmUmUfCfCfAmAmAmAmAmCmCmAmAmUm |
| | 50 | P1AmsUfsUmGmGmUfUmUmUmUmGmGmAmAfUmUfCmAmGmsUmsAm |
| siPKKa1-M2SP1 | 51 | CmsUmsGmAmAfUmUfCfCfAmAmAmAmAmCmCmAmAmUm |
| | 52 | P1AmsUfsUmGmGmUfUmUfUfUmGmGmAmAfUmUfCmAmGmsUmsAm |
| siPKKa1-M3SP1 | 53 | CmsUmsGmAmAfUmUfCfCfAmAmAmAmAmCmCmAmAmUm |
| | 54 | P1AmsUfsUmGmGmUfUmUmUmUmGmGmAmAfUmUfCmAmGmsUmsAm |
| siPKKa2-M1SP1 | 55 | UmsAmsCmUmGmAmAmUmUfCfCfAmAmAmAmAmCmCmAmAmUm |
| | 56 | P1AmsUfsUmGmGmUfUmUmUmUmGmGmAmAfUmUfCmAmGmUmAmsUmsAm |

(continued)

| siRNA No. | SEQ ID NO: | Sequence direction 5'-3' |
|---|---|---|
| siPKKa2-M2SP1 | 57 | UmsAmsCmUmGmAmAfUmUfCfCfAmAmAmAmAmCmCmAmAmUm |
| | 58 | P1AmsUfsUmGmGmUfUmUfUfUmGmGmAmAfUmUfCmAmGmUmAmsUmsAm |
| siPKKa2-M3SP1 | 59 | UmsAmsCmUmGmAmAfUmUfCfCfAmAmAmAmAmCmCmAmAmUm |
| | 60 | P1AmsUfsUmGmGmUfUmUmUmUmGmGmAmAfUmUfCmAmGmUmAmsUmsAm |

Wherein, capital letters C, G, U, and A indicate the base composition of the nucleotides; the lowercase m indicates that the nucleotide adjacent to the left side of the letter m is a methoxy modified nucleotide; the lowercase f indicates that the nucleotide adjacent to the left side of the letter f is a fluoro modified nucleotide; the lowercase letter s indicates that the two nucleotides adjacent to the left and right of the letter s are linked by phosphorothioate group; and P1 represents that the nucleotide adjacent to the right side of P1 is a 5'-phosphate nucleotide or a 5'-phosphate analogue modified nucleotide. In some embodiments, P1 represents specifically modified VP, Ps or P, wherein the letter combination VP represents that the nucleotide adjacent to the right side of the letter combination VP is a 5'-(E)-vinylphosphonate (E-VP) modified nucleotide, the letter combination Ps represents that the nucleotide adjacent to the right side of the letter combination Ps is a phosphorothioate modified nucleotide, and the capital letter P represents that the nucleotide adjacent to the right side of the letter P is a 5'-phosphate nucleotide.

In the siRNA or the siRNA conjugate of the present disclosure, each pair of adjacent nucleotides is linked via a phosphodiester bond or phosphorothioate diester bond. The non-bridging oxygen atom or sulfur atom in the phosphodiester bond or phosphorothioate diester bond is negatively charged, and may be present in the form of hydroxy or sulfhydryl. Moreover, the hydrogen ion in the hydroxy or sulfhydryl may be partially or completely substituted with a cation. The cation may be any cation, such as one of a metal cation, an ammonium ion $NH4^+$ or an organic ammonium cation. In order to increase solubility, in some embodiments, the cation is selected from one or more of an alkali metal ion, an ammonium cation formed by a tertiary amine and a quaternary ammonium cation. The alkali metal ion may be $K^+$ and/or $Na^+$, and the cation formed by the tertiary amine may be an ammonium ion formed by triethylamine and/or an ammonium ion formed by N,N-diisopropylethylamine. Thus, the siRNA or siRNA conjugate of the present disclosure may be at least partially present in the form of salt. In some embodiment, the non-bridging oxygen atom or sulfur atom in the phosphodiester bond or phosphorothioate diester bond at least partly binds to a sodium ion, and thus the siRNA or the siRNA conjugate of the present disclosure is present or partially present in the form of sodium salt.

Those skilled in the art clearly know that a modified nucleotide group may be introduced into the siRNA of the present disclosure by using a nucleoside monomer having a corresponding modification. The methods for preparing the nucleoside monomer having the corresponding modification and the methods for introducing the modified nucleotide group into the siRNA are also well-known to those skilled in the art. All the modified nucleoside monomers may be either commercially available or prepared by known methods.

Preparation of the siRNA conjugate of the present disclosure

The siRNA conjugate above may be synthesized by methods that have been described in detail in the prior art. For example, preparation methods of various siRNA conjugates are described in detail in WO2015006740A2. SiRNA conjugates of the present disclosure are obtained by a manner well known to those skilled in the art. For example, the preparation method of the structure shown in formula (305) is described in WO2014025805A1, and the preparation method of the structure shown in formula (307) is described by Rajeev et al. in ChemBioChem 2015, 16, 903-908. China patent application CN110959011A also discloses in detail the method for preparing the siRNA conjugate shown in formula (308). The contents of the above documents are hereby incorporated by reference in their entirety.

The siRNA conjugate of the present disclosure may also be used in combination with other pharmaceutically acceptable excipients, which may be one or more of the various conventional formulations or compounds in the art. For details, please refer to the above description of the pharmaceutical compositions of the present disclosure.

Use of the siRNA, the pharmaceutical composition and the siRNA conjugate of the present disclosure

In yet another aspect, the present disclosure provides the use of the siRNA and/or the pharmaceutical composition and/or the siRNA conjugate of the present disclosure in the manufacture of a medicament for treating and/or preventing an inflammatory disease or an embolic disease or a physiological condition, especially hereditary angioedema and related symptoms thereof.

In yet another aspect, the present disclosure provides a method for preventing and/or treating an inflammatory disease or an embolic disease or a physiological condition, especially hereditary angioedema and related symptoms thereof, wherein the method comprises administering an effective amount of the siRNA and/or the pharmaceutical composition and/or the siRNA conjugate of the present disclosure to a subject in need.

It is possible to achieve the purpose of preventing and/or treating the inflammatory disease or the embolic disease or the physiological condition, especially hereditary angioedema and related symptoms thereof through the mechanism of RNA interference by administering the active ingredients of the siRNA of the present disclosure to the subject in need. Thus, the siRNA and/or the pharmaceutical composition and/or the siRNA conjugate of the present disclosure may be used for preventing and/or treating the inflammatory disease or the embolic disease or the physiological condition, especially hereditary angioedema and related symptoms thereof, or for the manufacture of a medicament for preventing and/or treating or alleviating the inflammatory disease or the embolic disease or the physiological condition,

especially hereditary angioedema and related symptoms thereof.

In some embodiments, the inflammatory disease may be a chronic inflammatory disease or an acute inflammatory disease. In some embodiments, the inflammatory disease includes but is not limited to hereditary angioedema (HAE), edema, angioedema, swelling, angioedema of eyelid, eye edema, macular edema or cerebral edema. In some embodiments, the thrombembolia disease includes but is not limited to thrombus, thrombembolia, deep vein thrombosis, pulmonary embolism, myocardial infarction, apoplexy or infarction.

The diseases may have one or more risk factors, causes or consequences in common.

Some risk factors and causes for the development of the inflammatory diseases comprise genetic predisposition and environmental factors to the inflammatory disease. In some embodiments, the subject has a mutated complement 1 esterase inhibitor (C1-INH) gene or a mutated factor 12 (FXII) gene. In some embodiments, the subject carries or has an angiotensin-converting enzyme inhibitor (ACE inhibitor) or an angiotensin II receptor blocker (ARB). In some embodiments, the subject already has an allergic reaction leading to angioedema. In some embodiments, the subject suffers from HAE type I. In some embodiments, the subject suffers from HAE type II. In some embodiments, the subject suffers from HAE type III. Some results related to the development of inflammatory diseases comprise edema/swelling of various body parts comprising extremities (i.e., hands, feet, arms, legs), intestines (abdominal cavity), face, genitalia, throat (i.e., larynx); blood vessel permeability; blood vessel leakage; systemic inflammation; abdominal pain; flatulence; vomiting; diarrhea; itchy skin; respiratory (asthmatic) reaction; rhinitis; allergic reaction; bronchoconstriction; hypotension; coma and death.

Some risk factors and causes for the development of the thrombembolia diseases comprise genetic predisposition to the thrombembolia, surgery (specifically orthopedic surgery), malignant tumor, pregnancy, old age, use of oral contraceptives, atrial fibrillation, previous thromboembolic symptoms, chronic inflammatory diseases and hereditary or acquired prevention of thrombotic coagulation diseases. Some results related to the development of the thromboembolic symptoms comprise decreased blood flow through affected blood vessels, tissue death and death.

As used herein, the term "administration/administer" refers to the placement of the siRNA, the pharmaceutical composition, and/or the siRNA conjugate of the present disclosure into a body of a subject by a method or a route that at least partly locates the siRNA, the pharmaceutical composition, and/or the siRNA conjugate of the present disclosure at a desired site to produce a desired effect. Suitable administration routes for the methods of the present disclosure comprise topical administration and systemic administration. In general, the topical administration results in the delivery of more siRNA conjugate to a particular site compared with the systemic circulation of the subject; whereas the systemic administration results in the delivery of the siRNA, the pharmaceutical composition, and/or the siRNA conjugate of the present disclosure to the substantial systemic circulation of the subject. Considering that the present disclosure aims at providing a means for preventing and/or treating the inflammatory disease or the embolic disease or the physiological condition, especially hereditary angioedema and related symptoms thereof, in some embodiments, an administration mode capable of delivering drugs to liver is used.

The administration to a subject may be achieved by any suitable routes known in the art, including but not limited to, oral or parenteral route, such as intravenous administration, intramuscular administration, subcutaneous administration, transdermal administration, airway administration (aerosol), pulmonary administration, nasal administration, rectal administration and topical administration (including buccal administration and sublingual administration). The administration frequency may be once or more times daily, weekly, biweekly, triweekly, monthly, bimonthly, quarterly, semiannually or annually.

The dose of the siRNA, the pharmaceutical composition, or the siRNA conjugate of the present disclosure may be a conventional dose in the art, and the dose may be determined according to various parameters, especially age, weight and gender of a subject. Toxicity and efficacy may be measured in cell cultures or experimental animals by standard pharmaceutical procedures, for example, by determining $LD_{50}$ (the lethal dose that causes 50% population death), and $ED_{50}$ (the dose that can cause 50% of the maximum response intensity in a quantitative response, and that causes 50% of the experimental subjects to have a positive response in a qualitative response). The dose range for human may be derived based on the data obtained from cell culture analysis and animal studies.

When administrating the siRNA, the pharmaceutical composition or the siRNA conjugate of the present disclosure, for example, to male or female C57BL/6J mice or C3H/HeNCrlVr mice of 6-12 weeks old and 18-25 g body weight, and calculating based on the amount of the siRNA: (i) for the siRNA conjugate, the dosage of the siRNA thereof may be 0.001-100 mg/kg body weight, and in further embodiments, is 0.01-50 mg/kg body weight, and in some embodiments, is 0.05-20 mg/kg body weight, in some other embodiments is 0.1-15 mg/kg body weight, and in some other embodiments, is 0.1-10 mg/kg body weight; and (ii) for a pharmaceutical composition formed by an siRNA and a pharmaceutically acceptable carrier, the dosage of the siRNA thereof may be 0.001-50 mg/kg body weight, in some embodiments, is 0.01-10 mg/kg body weight, in some embodiments, is 0.05-5 mg/kg body weight, and in some embodiments, is 0.1-3 mg/kg body weight.

In addition, by introducing the siRNA and/or pharmaceutical composition and/or siRNA conjugate of the present disclosure into a cell, the purpose of inhibiting PKK gene expression in the cell can also be achieved through the

mechanism of RNA interference.

In the case where the expression of the PKK gene in the cell is inhibited by using the method provided by the present disclosure, the amount of the siRNA in the siRNA, the pharmaceutical composition, and/or the siRNA conjugate provided is typically: in an amount sufficient to reduce the expression of the target mRNA and result in an extracellular concentration of 1 pM to 1 μM, or 0.01 nM to 100 nM, or 0.05 nM to 50 nM or 0.05 nM to about 5 nM on the surface of the target cell. The amount required to achieve this local concentration will vary with various factors, including the delivery method, the delivery site, the number of cell layers between the delivery site and the target cells or tissues, the delivery route (topical or systemic), etc. The concentration at the delivery site may be significantly higher than that on the surface of the target cells or tissues.

Kit

In yet another aspect, the present disclosure provides a kit, wherein the kit comprises an effective amount of at least one of the siRNA, the pharmaceutical composition, and the siRNA conjugate of the present disclosure.

In some embodiments, the kit disclosed herein may provide the siRNA in a container. In some embodiments, the kit of the present disclosure may comprise a container providing pharmaceutically acceptable excipients. In some embodiments, the kit may further comprise additional ingredients, such as stabilizers or preservatives. In some embodiments, the kit herein may comprise at least one additional therapeutic agent in a container other than the container providing the siRNA herein. In some embodiments, the kit may comprise an instruction for mixing the siRNA with the pharmaceutically acceptable carrier and/or excipients or other ingredients (if present).

In the kit of the present disclosure, the siRNA and the pharmaceutically acceptable carrier and/or the excipients as well as the siRNA, the pharmaceutical composition, and/or the siRNA conjugate and/or the pharmaceutically acceptable excipients may be provided in any form, e.g., in a liquid form, a dry form, or a lyophilized form. In some embodiments, the siRNA and the pharmaceutically acceptable carrier and/or the excipients as well as the pharmaceutical composition and/or the siRNA conjugate and optional pharmaceutically acceptable excipients are substantially pure and/or sterile. In some embodiments, sterile water may be provided in the kit of the present disclosure.

Hereinafter, the present disclosure will be further described by examples, but is not limited thereto in any respect.

## Examples

Unless otherwise specified, the agents and culture media used in following examples are all commercially available, and the procedures used such as nucleic acid electrophoresis, real-time PCR are all performed according to methods described in Molecular Cloning (Cold Spring Harbor Laboratory Press (1989)).

C57BL/6J mice, purchased from SPF (Beijing) Biotechnology Co., Ltd., hereinafter referred to as C57 mice.

The Lipofectamine™2000 (Invitrogen) is used as the transfection reagent when cells are transfected with the siRNA and the siRNA conjugate for PKK gene synthesized in the present disclosure or the siRNA and the siRNA conjugate as negative controls, the specific operation refers to the instructions provided by the manufacturer.

Unless otherwise specified, ratios of reagents provided below are all calculated by volume ratio (v/v).

Preparation Example 1

Preparation of siRNA conjugate 1

In this preparation example, the siRNA conjugate 1 was synthesized. The siRNA conjugate was an siRNA conjugate formed after an L-9 conjugate molecule was conjugated with an siRNA numbered siPKKa1M1S. The sequence of the siRNA conjugated in this siRNA conjugate was shown in Table 2.

According to the preparation method of the conjugate 16 described in Preparation Example 14 of CN110959011A, the following siRNA conjugate 1 in Table 2 was prepared, with the only difference that a sense strand and an antisense strand of an siRNA contained in the siRNA conjugate 1 were shown in Table 2 respectively. The sense strand and the antisense strand of the siRNA were respectively synthetized according to a nucleic acid sequence of the siRNA numbered L10-siPKKa1M1S in the Table 2 below. The siRNA conjugate 1 was diluted to a concentration of 0.2 mg/mL (calculated based on siRNA) with ultra-pure water (prepared by Milli-Q ultra-pure water instrument, with resistivity of 18.2 MQ*cm (25°C)). The molecular weight was measured by Liquid Chromatography-Mass Spectrometry (LC-MS, purchased from Waters Corp., model: LCT Premier). Specifically,
the molecular weight of the siRNA conjugate 1: a theoretical value of the sense strand was 7,507.51, and a measured value of the sense strand was 7,506.73, a theoretical value of the antisense strand was 6,987.64 and a measured value of the antisense strand was 6,987.56.

The measured value was consistent with the theoretical value, showing that the synthesized siRNA conjugate

1 was a target designed double-stranded nucleic acid sequence. The siRNA conjugate 1 had the structure as shown in Formula (403), and the siRNA contained in the siRNA conjugate had an siRNA sequence corresponding to the siRNA conjugate 1 in Table 2.

Table 2 siRNA conjugate

| Preparation Example No. | siRNA conjugate No. | siRNA | Sequence direction 5'-3' | | SEQ ID NO |
|---|---|---|---|---|---|
| Preparation Example 1 | siRNA conjugate 1 | siPKKa 1 M1S | Sense strand | CmsUmsGmAmAmUmUfCfCfAmAmAmAmAmCmCmAmAmUm | 61 |
| | | | Antisense strand | AmsUfsUmGmGmUfUmUmUmUmGmGmAmAfUmUfCmAmGmsUmsAm | 62 |

Capital letters C, G, U, and A indicate the base composition of the nucleotides; the lowercase m indicates that the nucleotide adjacent to the left side of the letter m is a methoxy modified nucleotide; the lowercase f indicates that the nucleotide adjacent to the left side of the letter f is a fluoro modified nucleotide; and the lowercase letter s indicates that the two nucleotides adjacent to the left and right of the letter s are linked by phosphorothioate group.

Preparation Examples 2-5

Synthesis of the siRNAs provided by the present disclosure

The sense strands or the antisense strands of the siRNA sequences listed in Table 3 were respectively synthesized by a regular solid phase synthesis method, and DEPC water was used to dissolve the mutually complementary sense strands and antisense strands in equimolar in Table 3, and then followed by annealing to obtain the following siRNAs provided by the present disclosure, which were respectively denoted with siPKKa1M1S, siPKKa2M4, siPKKa and siPKKa3. The sequences of the above siRNAs were shown in Table 3.

Comparative Preparation Example 1

Synthesis of reference siRNA

The sense strand and antisense strand corresponding to the siRNA numbered NC in Table 3 were respectively synthesized by solid phase synthesis. The obtained equimolar sense strand and antisense strand were dissolved respectively with DEPC water, and then annealed to obtain the reference siRNA numbered NC. The NC siRNA was a double-stranded oligonucleotide chain that did not target any known human, mouse or rat genes.

Table 3 siRNA sequences

| Preparation Example No. | siRNA | Sequence direction 5'-3' | | SEQ ID NO |
|---|---|---|---|---|
| Preparation Example 2 | siPKKa1M1S | Sense strand | CmsUmsGmAmAmUmUfCfCfAmAmAmAmAmCmCmAmAmUm | 63 |
| | | Antisense strand | AmsUfsUmGmGmUfUmUmUmUmGmGmAmAfUmUfCmAmGmsUmsAm | 64 |

(continued)

| Preparation Example No. | siRNA | Sequence direction 5'-3' | | SEQ ID NO |
|---|---|---|---|---|
| Preparation Example 3 | siPKKa2M4 | Sense strand | UmACmUGmAAmUUfCfCfAAmAAmACmCAmAUm | 75 |
| | | Antisense strand | AUfUGmGUfUUmUUmGGmAAfUUfCAmGUmAUmA | 76 |
| Preparation Example 4 | siPKKa | Sense strand | CUGAAUUCCAAAAACCAAU | 77 |
| | | Antisense strand | AUUGGUUUUUGGAAUUCAG | 78 |
| Preparation Example 5 | siPKKa3 | Sense strand | UACUGAAUUCCAAAAACCAAA | 79 |
| | | Antisense strand | UUUGGUUUUUGGAAUUCAGUAUA | 80 |
| Comparative Preparation Example 1 | NC | Sense strand | UUCUCCGAACGUGUCACGU | 65 |
| | | Antisense strand | ACGUGACACGUUCGGAGAA | 66 |

Capital letters C, G, U, and A indicate the base composition of the nucleotides; the lowercase m indicates that the nucleotide adjacent to the left side of the letter m is a methoxy modified nucleotide; the lowercase f indicates that the nucleotide adjacent to the left side of the letter f is a fluoro modified nucleotide; the lowercase letter s indicates that the two nucleotides adjacent to the left and right of the letter s are linked by phosphorothioate group.

According to the method of Preparation Example 1, the molecular weights of the siRNAs above were detected respectively.

The molecular weight of the Preparation Example 2: a theoretical value of the sense strand was 6,259.23, and a measured value of the sense strand was 6,259.01, a theoretical value of the antisense strand was 6,987.64 and a measured value of the antisense strand was 6,987.56.

The molecular weight of the Preparation Example 3: a theoretical value of the sense strand was 6,764.30, and a measured value of the sense strand was 6,764.12, a theoretical value of the antisense strand was 7,418.50 and a measured value of the antisense strand was 7,418.96.

The molecular weight of the Preparation Example 4: a theoretical value of the sense strand was 5,996.70, and a measured value of the sense strand was 5,996.33, a theoretical value of the antisense strand was 6,041.59 and a measured value of the antisense strand was 6,041.22.

The molecular weight of the Preparation Example 5: a theoretical value of the sense strand was 6,655.12, and a measured value of the sense strand was 6,655.02, a theoretical value of the antisense strand was 7,289.30 and a measured value of the antisense strand was 7,289.11.

The measured values were consistent with the theoretical values, and it was confirmed that the obtained siRNAs had sequences corresponding to the siRNAs shown in Table 3 respectively.

After the siRNAs or siRNA conjugates of the present disclosure above were prepared, the siRNAs or siRNA conjugates were freeze-dried into solid powder for later use.

Experimental Example 1

Determination of inhibitory percentage of siRNA provided by the present disclosure on PKK mRNA in primary hepatocytes of mice

Primary hepatocytes of mice were extracted from fresh liver tissues of C57BL/6J mice, inoculated into tissue culture dishes coated with type I collagen, and cultured in RPMI 1640 media containing 1 x double-antibody and 10%

FBS at 37°C, and cultured in an incubator containing 5% $CO_2$/95% air for 30 minutes.

The culture media were discarded, and the density of the primary hepatocytes of mice was adjusted to 2 × $10^5$ cells/mL by opti-MEM, to obtain the suspension of the primary hepatocytes of mice. Then, the suspension of the primary hepatocytes of mice obtained was added into different culture wells of a 12-well plate, and the primary hepatocytes of mice were inoculated into the culture wells. The volume of the added suspension of the primary hepatocytes of mice was 1 mL/well, and the number of the primary hepatocytes of mice was 2 × $10^5$ cells/well.

DEPC water was used to prepare the siRNAs obtained in the Preparation Examples 2-5 and Comparative Preparation Example 1 into siRNA working fluids with a concentration of 50 μM respectively.

A 1A solution was prepared. For each siRNA, 1A1-1A3 solutions were prepared respectively, and each part of the 1A1-1A3 solutions contained 1.1 μl of the siRNA working fluid above and 50 μl of Opti-MEM media in turn.

A 1B solution was prepared, and each part of the 1B solution contained 1 μl of Lipofectamine™ 2000 and 50 μl of Opti-MEM media.

One part of the 1B solution was respectively mixed with one part of the 1A solution of each siRNA obtained, and incubated at room temperature for 20 minutes to obtain a transfection complex 1X of each siRNA.

One part of the 1B solution was mixed with 50 μl of Opti-MEM media and incubated at room temperature for 20 minutes to obtain a transfection complex 1X'.

The transfection complex 1X of each siRNA was respectively added in the culture well, and evenly mixed, with an addition amount of 100 μl/well, to obtain a transfection complex containing the siRNA with the final concentration of the siRNA about 50 nM. The transfection complex 1X of each siRNA was respectively transfected with three culture wells to obtain a transfection mixture containing the siRNA, which was designated as the test group.

The transfection complex 1X' was respectively added into another three culture wells with an addition amount of 100 μl/well, to obtain a transfection mixture not containing the siRNA, which was designated as a blank control group.

Each transfection mixture containing the siRNA and the transfection mixture not containing the siRNA were respectively transfected in culture wells for 4 hours, and then 1 ml of H-DMEM complete media containing 20% FBS was added to each well. The 12-well plate was placed in a $CO_2$ incubator to continuously culture at 37°C for 24 hours.

Then, RNAVzol (purchased from Vigorous Biotechnology Beijing Co., Ltd., article number N002) was used to respectively extract the total RNA from the cells in each well according to the methods described in the instructions.

For the cells in each well, 1 μg of the total RNA was taken, and a reagent provided by a reverse transcription kit Goldenstar™ RT6 cDNA Synthesis Kit (purchased from Beijing Tsingke Biotechnology Co., Ltd., article number TSK301M) was used, wherein Goldenstar™ Oligo $(dT)_{17}$ was selected as the primer, and 20 μl of reverse transcription reaction system was configured according to the reverse transcription operation steps in the kit manual to reverse the total RNA of the cells in each well. The conditions for reverse transcription were as follows: for each reverse transcription reaction system, the reverse transcription reaction system was incubated at 50°C for 50 minutes, then incubated at 85°C for 5 minutes, and finally incubated at 4°C for 30 seconds. After the reaction, 80 μl of DEPC water was added to the reverse transcription reaction system to obtain a solution containing cDNA.

For each reverse transcription reaction system, 5 μl of the solution containing cDNA was taken as the template, and 20 μl of qPCR reaction system was prepared by using the reagent provided by NovoStart® SYBR qPCR SuperMix Plus Kit (purchased from Novoprotein Science and Technology Co., Ltd., article No. E096-01B), wherein the PCR primer sequences for amplifying the target gene PKK and internal reference gene GAPDH were shown in Table 4, and the final concentration of each primer was 0.25 μM. Each qPCR reaction system was placed on ABI StepOnePlus Real-Time PCR instrument, and amplified by three-step method. The amplification procedure was pre-denatured at 95°C for 10 minutes, then denatured at 95°C for 30 seconds, annealed at 60°C for 30 seconds, and extended at 72°C for 30 seconds. After repeating the above denaturation, annealing and extension processes for 35 times, a product W1 containing the amplified target gene PKK and internal reference gene GAPDH was obtained. The product W 1 was incubated at 95°C for 15 seconds, 60°C for 1 minute and 95°C for 15 seconds in turn. The dissolution curves of the target gene PKK and the internal reference gene GAPDH in the product W1 were collected by real-time fluorescence quantitative PCR, and the Ct values of the target gene PKK and the internal reference gene GAPDH were obtained.

Table 4 Primer information

| Name of gene | Type of primer | Nucleotide sequence (5'-3') | SEQ ID NO |
|---|---|---|---|
| PKK in mice | Upstream primer | TGCCTTCCAAAGCTGACACA | 67 |
| | Downstream primer | GTGATACCCACCAACTGCCA | 68 |
| GAPDH in mice | Upstream primer | AACTTTGGCATTGTGGAAGGGCTC | 69 |
| | Downstream primer | TGGAAGAGTGGGAGTTGCTGTTGA | 70 |

Comparative Ct ($\Delta\Delta$Ct) method was used to calculate relative quantitative expression of the target gene PKK in each test group and the control group. The calculation method was as follows:

$$\Delta Ct \text{ (test group)} = Ct \text{ (target gene of test group)} - Ct \text{ (internal reference gene of test group)}$$

$$\Delta Ct \text{ (control group)} = Ct \text{ (target gene of control group)} - Ct \text{ (internal reference gene of control group)}$$

$$\Delta\Delta Ct \text{ (test group)} = \Delta Ct \text{ (test group)} - \Delta Ct \text{ (mean value of control group)}$$

$$\Delta\Delta Ct \text{ (control group)} = \Delta Ct \text{ (control group)} - \Delta Ct \text{ (mean value of control group)}$$

$\Delta$Ct (mean value of control group) was the arithmetic mean value of $\Delta$Ct (control group) of each of the three culture wells of the control group. Therefore, each culture well of the test group and the control group corresponded to one $\Delta\Delta$Ct value.

On the basis of the control group, the expression level of PKK mRNA in the test group was normalized, and the expression level of PKK mRNA in the blank control group was defined as 100%.

$$\text{The relative expression level of PKK mRNA in the test group} = 2^{-\Delta\Delta Ct(\text{test group})} \times 100\%.$$

$$\text{The inhibition percentage of test group} = (1\text{-relative expression level of PKK mRNA in the test group}) \times 100\%.$$

The inhibition percentage to PKK mRNA of each siRNA was summarized in Table 5. For the siRNAs of the same test group, the inhibition percentage to the PKK mRNA was the arithmetic mean value of the inhibition percentage of the test group to PKK mRNA determined through the three culture wells.

Table 5 Inhibition to PKK mRNA in primary hepatocytes of mice

| Preparation Example | siRNA | Relative expression level of PKK mRNA, % | Inhibition percentage to PKK mRNA % |
|---|---|---|---|
| Preparation Example 2 | siPKKa1M1S | 27.7 | 72.3 |
| Preparation Example 3 | siPKKa2M4 | 34.4 | 65.6 |
| Preparation Example 4 | siPKKa | 40.0 | 60.0 |
| Preparation Example 5 | siPKKa3 | 26.2 | 73.8 |
| Comparative Preparation Example 1 | NC | 107.6 | -7.6 |

From the results in Table 5, it can be seen that the siRNA provided by the present disclosure shows high PKK mRNA inhibitory activity in the primary hepatocytes of mice under the conditions of different lengths, mismatches between antisense strand and mRNA or different modifications contained in the siRNAs. For the siRNA with a concentration of 50 nM, the inhibition percentages of Preparation Examples 2-5 on PKK mRNA are all higher than 60.0%, and the inhibition percentage of Preparation Example 2 on PKK mRNA reaches 72.3%.

Experimental Example 2

Activity determination of siRNA conjugates provided by the present disclosure in C57BL/6J mice (*in vivo*)

C57BL/6J mice were randomly divided into groups (all males) with five mice in each group and each group was given different doses of siRNA conjugate 1. Dosages for all animals were calculated according to body weights thereof, and were given once by subcutaneous injection. The siRNA conjugate 1 was given in the form of 0.1 mg/ml and 0.3 mg/ml of 0.9 wt% sodium chloride aqueous solution, and an administration volume was 10 ml/kg of mouse body weight, i.e., the dosages of the siRNA conjugate 1 were 1 mg/kg and 3 mg/kg respectively. In addition, 10 male C57BL/6J mice were used as the control group and given saline, which was 0.9 wt% sodium chloride aqueous solution, and the administration volume was 10 ml/kg mouse body weight. On the $8^{th}$ day after administration, the mice were killed, plasma was collected, and 3.2 wt% (0.109 mol/L) sodium citrate dihydrate aqueous solution was added in a volume ratio of anticoagulant to plasma of 1: 9 (v/v) to prevent coagulation, and the plasma was centrifuged.

About 100 mg of the left large lobe of liver/mice was taken and preserved with RNA later (Sigma Aldrich Company). Then, for the liver tissue of each mouse, the liver tissues were respectively homogenated with a tissue homogenizer, and the total RNA of the liver tissue of each mouse was extracted with Trizol (Thermo Fisher Company) according to the operation steps described in the instructions. The extracted total RNA was reverse transcribed by using ImProm-II™ reverse transcription kit (Promega Company) according to the instructions thereof. The conditions for reverse transcription were as follows: the reverse transcription reaction system was incubated at 50°C for 50 minutes, then incubated at 85°C for 5 minutes, and finally incubated at 4°C for 30 seconds. After the reaction, 80 μl of DEPC water was added to the reverse transcription reaction system to obtain a solution containing cDNA. Then, the expression of PKK mRNA in the liver tissue was detected by fluorescence quantitative PCR kit (Beijing CoWin Biosciences).

For each reverse transcription reaction system, 5 μl of the solution containing cDNA was taken as the template, and 20 μl of qPCR reaction system was prepared by using the reagent provided by NovoStart® SYBR qPCR SuperMix Plus Kit (purchased from Novoprotein Science and Technology Co., Ltd., article No. E096-01B), wherein the PCR primer sequences for amplifying the target gene PKK and internal reference gene GAPDH were shown in Table 6, and the final concentration of each primer was 0.25 μM. Each qPCR reaction system was placed on ABI StepOnePlus Real-Time PCR instrument, and amplified by three-step method. The amplification procedure was pre-denatured at 95°C for 10 minutes, then denatured at 95°C for 30 seconds, annealed at 60°C for 30 seconds, and extended at 72°C for 30 seconds. After repeating the above denaturation, annealing and extension processes for 40 times, a product W2 containing the amplified target gene PKK and internal reference gene GAPDH was obtained. The product W2 was incubated at 95°C for 15 seconds, 60°C for 1 minute and 95°C for 15 seconds in turn. The dissolution curves of the target gene PKK and the internal reference gene GAPDH in the product W2 were collected by real-time fluorescence quantitative PCR, and the Ct values of the target gene PKK and the internal reference gene GAPDH were obtained.

Table 6 Detection of primer sequences

| Name of gene | Type of primer | Nucleotide sequence (5'-3') | SEQ ID NO |
|---|---|---|---|
| PKK in mice | Upstream primer | TGCACCACCAACTGCTTAG | 71 |
| | Downstrea m primer | GGATGCAGGGATGATGTTC | 72 |
| GAPDH in mice | Upstream primer | TGCCTTCCAAAGCTGACACA | 73 |
| | Downstream primer | GTGATACCCACCAACTGCCA | 74 |

Comparative Ct (ΔΔCt) method was used to calculate relative quantitative expression of the target gene PKK in each of the test group and the control group. The calculation method was as follows:

$$\Delta Ct\ (\text{test group}) = Ct\ (\text{target gene of test group}) - Ct\ (\text{internal reference gene of test group})$$

$$\Delta Ct\ (\text{control group}) = Ct\ (\text{target gene of control group}) - Ct\ (\text{internal reference gene of control group})$$

$$\Delta\Delta Ct\ (\text{test group}) = \Delta Ct\ (\text{test group}) - \Delta Ct\ (\text{mean value of control group})$$

$$\Delta\Delta Ct \text{ (control group)} = \Delta Ct \text{ (control group)} - \Delta Ct \text{ (mean value of control group)}$$

ΔCt (mean value of control group) was the arithmetic mean value of ΔCt (control group) of each of the 10 mice of the control group. Therefore, each test mouse of the test group and the control group corresponded to one ΔΔCt value.
On the basis of the control group, the expression level of PKK mRNA in the test group was normalized, and the expression level of PKK mRNA in the saline control group was defined as 100%.

$$\text{The relative expression level of PKK mRNA in the test group} = 2^{-\Delta\Delta Ct(\text{test group})} \times 100\%.$$

For the siRNAs of the same test group, the mean value of the relative expression level of the PKK mRNA of the test group under each concentration was the arithmetic mean value of the relative expression level of the five mice at the concentration. Graphpad prism 6.0 statistical analysis software was used for data analysis. The expression amount of PKK mRNA in the control group was denoted as 100%, and accordingly, the inhibition percentage to the expression amount of the PKK mRNA was denoted as 0%. The test results were normalized by the expression amount of the PKK mRNA in the control group, and the results were shown in Table 7. In Table 7, the dosage was calculated on the basis of the siRNA, the control group was not given with the siRNA, and the column of the corresponding dosage was marked with "-". The inhibition percentage to the PKK mRNA was the average value of the inhibition percentages of one group of mice given the corresponding siRNA conjugate to the PKK mRNA:

$$\text{Inhibition percentage to PKK mRNA} = (1 - \text{relative expression level of PKK mRNA}) \times 100\%.$$

Table 7 Inhibition percentage of the siRNA conjugate of the present disclosure to PKK mRNA in mice

| Preparation Example No. | siRNA conjugate No. | Dosage mg/kg | Inhibition percentage to PKK mRNA % |
|---|---|---|---|
| Preparation Example 1 | siRNA conjugate 1 | 1 | 51.68 |
| Preparation Example 1 | siRNA conjugate 1 | 3 | 81.31 |
| Control | Saline | - | 0 |

The results of the relative expression level of PKK mRNA in vivo after administration of different doses of siRNA conjugates were shown in Figure 1.
From the results in Table 7 and Figure 1, it can be seen that the siRNA conjugate 1 provided by the present disclosure shows better PKK mRNA inhibitory effects in the C57BL/6J mice. Relative to the saline of the control group, the inhibition percentage to PKK mRNA can reach 81.31% when the dosage of the siRNA conjugate 1 is 3 mg/kg.

Experimental Example 3

Study on the efficacy of the siRNA conjugate provided by the present disclosure on carrageenan-induced mouse foot swelling model

Carrageenan was a mucopolysaccharide from a cell wall of red algae, which had many types, wherein λ-carrageenan may be in a non-gel state at room temperature, which could cause inflammatory reactions after subcutaneous injection, such as edema, increased capillary permeability, hyperalgesia and erythema, and was used to induce rodent foot swelling models. In this experimental example, the mice were given different doses of the siRNA conjugate 1 and positive controls indomethacin and icatibant, and then the mouse foot swelling was induced by λ-carrageenan. A degree of inhibition to the increase of foot swelling by the siRNA conjugate provided by the present disclosure was determined by a drainage method, and the expression level of PKK mRNA in mice was detected by fluorescence quantitative PCR.
In this experiment, the physiological saline was a 0.9 wt% sodium chloride solution, which was purchased from Jiangxi Kelun Pharmaceutical Co., Ltd. (batch number C19070906). Normal saline was used to prepare the siRNA conjugate 1 obtained in the Preparation Example 1 into aqueous solutions with concentrations of 0.2 mg/ml, 0.6 mg/ml

and 1.8 mg/ml respectively. The positive controls indomethacin was purchased from Sigma-Aldrich Company (article number I7378, batch number WXBC4210V), and 0.5 wt% CMC-Na (sodium carboxymethylcellulose, purchased from Sinopharm Chemical Reagent Co., Ltd., article number 30036358, batch number 20180412) was used to prepare 2 mg/ml solution. The positive control icatibant acetate was purchased from MedChemExpress Company (article number HY-108896, batch number 81711), and was prepared into 1.2 mg/ml solution with Normal saline. Indomethacin was a non-steroidal anti-inflammatory drug, which could relieve swelling and pain of joints and soft tissues. Icatibant acetate was a powerful and selective competitive antagonist of bradykinin type 2 (B2) receptor, which was used to treat acute onset of hereditary angioedema (HAE) in adults, adolescents and children over 2 years old.

Thirty male ICR mice (purchased from Shanghai Lingchang Biotechnology Co., Ltd.) were randomly divided into 6 groups according to their body weights, with 5 mice in each group, which were labeled as G1-G6 groups respectively. The dosage of all animals was calculated according to their body weights, and the dosage volume of mice injected subcutaneously or gavage was 5 ml/kg of mouse body weight. The dosage solutions of mice in each group were as follows: The mice in Group G1 were injected with saline subcutaneously 7 days before the injection of k-carrageenan solution (marked as D-7), and the dosage volume was 5 ml/kg of mouse body weight.

The mice in Group G2 were injected with indomethacin by gavage one hour before the injection of k-carrageenan solution, and the dosage volume was 10 mg/kg of mouse body weight.

The mice in Group G3 were injected with icatibant subcutaneously one hour before the injection of k-carrageenan solution, and the dosage volume was 6 mg/kg of mouse body weight.

The mice in G4-G6 groups were injected with different doses of the siRNA conjugate 1 subcutaneously 7 days before the injection of k-carrageenan solution, and the dosages were 1 mg/kg, 3 mg/kg and 9 mg/kg respectively.

The λ-carrageenan solution was subcutaneously injected into right hind leg pads of the mice injected with different drugs in the above groups to induce foot swelling in mice. The λ-carrageenan, purchased from Sigma-Aldrich Company (Article No.22049), was prepared into 1 wt% solution with normal saline, and the injection volume was 40 μL/mouse. The day when the λ-carrageenan solution was injected was regarded as the first day of the experiment (denoted as D1).

One hour before injection, two hours, four hours and six hours after injection, the swelling degrees of hind limbs and toes of each animal were measured by a drainage method, wherein the measurement method was as follows: 70 ml of distilled water were injected into a 100 ml beaker, and zero calibration was carried out on an electronic balance. Before the test, a measuring line was marked at an ankle joint of the rat foot. The mouse was fixed, the toes of the hind limb of the mouse were put into the beaker, and when a horizontal plane overlapped with the measuring mark at the ankle joint of the mouse foot, the results on a display screen of the electronic balance were read. The reading gram (g) was a drainage volume (ml) of the toes of the mice after immersion in water, which indicated the foot swelling degree of the mice. Every time the same mouse was measured, the toes of the hind limb thereof were immersed in distilled water in the beaker at the same position, i.e., the measuring line marked at the ankle joint overlapped with the horizontal plane. Zero calibration was carried out again after each measurement.

The foot swelling degree, the maximum swelling degree increase and the time to reach the maximum swelling degree of the animals in each group were shown in Table 8. In Table 8, the maximum swelling degree increase of a certain group = (the maximum swelling degree of the group - the swelling degree of the group one hour before injection)/the swelling degree of the group one hour before injection × 100%. The time to reach the maximum swelling degree of a certain group referred to the time to reach the maximum swelling degree after injecting λ-carrageenan solution, and the maximum swelling degree referred to the maximum value of the swelling degree of the animals in a certain group.

After the last measurement of foot swelling, the animals were anesthetized by inhaling 2-5 v/v% isoflurane (purchased from Shenzhen RWD Technology Co., Ltd., batch number: 20120301). Afterwards, by inhaling excessive carbon dioxide, the animals were euthanized by cervical vertebra removal. For each mouse, 100 mg of the left lobe of the liver was taken, cut into 2 × 2 mm pieces, and preserved with RNA later (Sigma Aldrich Company). The expression level of PKK mRNA in the liver tissue was detected by the same method as in Experimental Example 2. The inhibition rate to PKK mRNA in the carrageenan-induced foot swelling model mice was shown in Table 9.

During the whole experiment, the animals did not have any abnormal appearance or behavior, and there was no significant difference among the groups.

Table 8 Foot swelling degree of animals in each group (g)

| Group | Drug | | 1 h before injection | 2 h after injection | 4 h after injection | 6 h after injection | Maxium swelling increase % | Time to reach the maximum swelling degree (h) |
|---|---|---|---|---|---|---|---|---|
| G1 | Saline | Average value | 0.17 | 0.26 | 0.29 | 0.24 | 70.6 | 4 |
| | | Standard deviation | 0.03 | 0.02 | 0.01 | 0.03 | | |
| G2 | Indomethacin | Average value | 0.20 | 0.24 | 0.25 | 0.23 | 25.0 | 4 |
| | | Standard deviation | 0.02 | 0.02 | 0.02 | 0.01 | | |
| G3 | Icatibant | Average value | 0.19 | 0.21 | 0.24 | 0.24 | 26.3 | 4 |
| | | Standard deviation | 0.03 | 0.01 | 0.01 | 0.01 | | |
| G4 | siRNA conjugate 1 (1 mg/kg) | Average value | 0.18 | 0.25 | 0.24 | 0.23 | 38.9 | 2 |
| | | Standard deviation | 0.01 | 0.01 | 0.01 | 0.01 | | |
| G5 | siRNA conjugate 1 (3 mg/kg) | Average value | 0.18 | 0.25 | 0.24 | 0.23 | 38.9 | 2 |
| | | Standard deviation | 0.01 | 0.01 | 0.01 | 0.02 | | |
| G6 | siRNA conjugate 1 (9 mg/kg) | Average value | 0.19 | 0.24 | 0.23 | 0.23 | 26.3 | 2 |
| | | Standard deviation | 0.01 | 0.01 | 0.01 | 0.01 | | |

As can be seen from Table 8, at 2 hours, 4 hours and 6 hours after the injection of the λ-carrageenan solution, the mice in G1-G6 groups have different degrees of foot swelling compared with those before the injection. In the G1 group injected with the normal saline, the maximum swelling degree increase reaches 70.6%, while in the G2-G6 group injected with different drugs, the maximum swelling degree increase is greatly reduced to below 39%. On the other hand, the time to reach the maximum swelling degree for all the mice in the G4-G6 groups injected with different doses of siRNA conjugate 1 of the present disclosure is short, which is 2 hours, and then the swelling degree is weakened, and the swelling inhibition degree is also obviously dose-related, which shows that the siRNA conjugate provided by the present disclosure has obvious and quick effect on inhibiting swelling in mice.

Compared with the other two positive control drugs (G2-G3 group), the siRNA conjugate provided by the present disclosure has similar or better effects.

Table 9 Inhibition percentage to PKK mRNA in animals of each group

| Group | Drug | Dosage | Inhibition percentage to PKK mRNA % (Average value) |
|---|---|---|---|
| G1 | Saline | -- | 0.00 |
| G2 | Indomethacin | 10 mg/kg | -3.55 |
| G3 | Icatibant | 6 mg/kg | 6.50 |
| G4 | siRNA conjugate 1 | 1 mg/kg | 67.36 |
| G5 | siRNA conjugate 1 | 3 mg/kg | 77.47 |
| G6 | siRNA conjugate 1 | 9 mg/kg | 82.43 |

From Table 9, it can be seen that, in the carrageenan-induced foot swelling model mice, the siRNA conjugate 1 provided by the present disclosure shows better PKK mRNA inhibitory effects in mice. Relative to the G1 saline group, the inhibition percentage of the siRNA conjugate 1 to PKK mRNA can reach more than 60%, wherein the inhibition percentage to PKK mRNA can reach 77.47% when the dosage is 3 mg/kg. When the dosage is 9 mg/kg, the inhibition percentage to PKK mRNA can reach 82.43%, showing obvious dose-dependent inhibition. However, the positive control groups G2-G3 do not significantly inhibit the expression level of PKK mRNA. It can be seen that the siRNA conjugate 1 of the present disclosure significantly inhibits the foot swelling increase by inhibiting the expression level of PKK mRNA, and has a good application prospect.

Some embodiments of the present disclosure are described in detail above, but the present disclosure is not limited to the specific details of the above-described embodiments. Various simple variations of the technical solution of the present disclosure can be made within the scope of the technical concept of the present disclosure, and these simple variations are within the protection scope of the present disclosure.

In addition, it is to be noted that each of the specific technical features described in the above embodiments can be combined in any suitable manner as long as no contradiction is caused. In order to avoid unnecessary repetition, the various possible combination manners are no longer described in the present disclosure.

In addition, the various different embodiments of the present disclosure may also be carried out in any combination as long as it does not contravene the idea of the present disclosure, which should also be regarded as the disclosure of the present disclosure.

## Claims

**1.** An siRNA, wherein the siRNA comprises a sense strand and an antisense strand, and each nucleotide in the siRNA is independently a modified or unmodified nucleotide, wherein the sense strand comprises a nucleotide sequence I, and the antisense strand comprises a nucleotide sequence II; the nucleotide sequence I and the nucleotide sequence II are at least partly reverse complementary to form a double-stranded region, wherein the nucleotide sequence I has the same length as and no more than three nucleotide differences from the nucleotide sequence shown in SEQ ID NO: 1; and the nucleotide sequence II has the same length as and no more than three nucleotide differences from the nucleotide sequence shown in SEQ ID NO: 2:

5'-CUGAAUUCCAAAAACCAAZi-3' (SEQ ID NO: 1);
5'-$Z_2$UUGGUUUUUGGAAUUCAG-3' (SEQ ID NO: 2),
wherein, $Z_1$ is U, and $Z_2$ is A; and
the nucleotide sequence I comprises a nucleotide $Z_3$ at a corresponding site to $Z_1$, the nucleotide sequence II

comprises a nucleotide $Z_4$ at a corresponding site to $Z_2$, and $Z_4$ is the first nucleotide from the 5' terminal of the antisense strand.

**2.** The siRNA according to claim 1, wherein the nucleotide sequence I has no more than one nucleotide difference from the nucleotide sequence shown in SEQ ID NO: 1, and/or the nucleotide sequence II has no more than one nucleotide difference from the nucleotide sequence shown in SEQ ID NO: 2;

preferably, the nucleotide difference between the nucleotide sequence II and the nucleotide sequence shown in SEQ ID NO: 2 comprises a difference at the position of $Z_4$, and $Z_4$ is selected from U, C or G; and
preferably, $Z_3$ is a nucleotide complementary to $Z_4$.

**3.** The siRNA according to claim 1 or 2, wherein the nucleotide sequence I is basically reverse complementary or substantially reverse complementary or completely reverse complementary to the nucleotide sequence II; the basically reverse complementary refers to no more than three base mispairings between two nucleotide sequences, the substantially reverse complementary refers to no more than one base mispairing between two nucleotide sequences; and the completely reverse complementary refers to no mispairing between two nucleotide sequences.

**4.** The siRNA according to any one of claims 1-3, wherein the nucleotide sequence I is the nucleotide sequence shown in SEQ ID NO: 3, and the nucleotide sequence II is the nucleotide sequence shown in SEQ ID NO: 4:

5'-CUGAAUUCCAAAAACCAA$Z_3$-3' (SEQ ID NO: 3);
5'-$Z_4$UUGGUUUUUGGAAUUCAG-3' (SEQ ID NO: 4),
wherein, $Z_3$ is selected from A, U, G or C; and $Z_4$ is a nucleotide complementary to $Z_3$; and
preferably, $Z_3$ is U, and $Z_4$ is A.

**5.** The siRNA according to any one of claims 1-4, wherein the sense strand further comprises a nucleotide sequence III, the antisense strand further comprises a nucleotide sequence IV, the nucleotide sequence III and the nucleotide sequence IV each independently have a length of 1-4 nucleotides, the nucleotide sequence III is linked to a 5' terminal of the nucleotide sequence I, the nucleotide sequence IV is linked to a 3' terminal of the nucleotide sequence II, and the nucleotide sequence III has the same length and is substantially reverse complementary or completely reverse complementary to the nucleotide sequence IV; the substantially reverse complementary refers to no more than one base mispairing between two nucleotide sequences; and the completely reverse complementary refers to no mispairing between two nucleotide sequences.

**6.** The siRNA according to claim 5, wherein the nucleotide sequences III and IV both have a length of one nucleotide, and the base of the nucleotide sequence III is A;

or, the nucleotide sequences III and IV both have a length of two nucleotides, and in the direction from the 5' terminal to the 3' terminal, the base composition of the nucleotide sequence III is UA;
or, the nucleotide sequences III and IV both have a length of three nucleotides, and in the direction from the 5' terminal to the 3' terminal, the base composition of the nucleotide sequence III is AUA;
or, the nucleotide sequences III and IV both have a length of four nucleotides, and in the direction from the 5' terminal to the 3' terminal, the base composition of the nucleotide sequence III is UAUA.

**7.** The siRNA according to any one of claims 1-6, wherein the antisense strand further comprises a nucleotide sequence V, which has a length of 1-3 nucleotides and is linked to 3' terminal of the antisense strand, thereby constituting a 3' overhang of the antisense strand;.

or, the nucleotide sequence V has a length of two nucleotides;
or, the nucleotide sequence V is two continuous thymidine deoxyribonucleotides or two continuous uridine ribonucleotides; or the nucleotide sequence V is complementary to a nucleotide at a corresponding site of a target mRNA.

**8.** The siRNA according to any one of claims 1-7, wherein the sense strand of the siRNA comprises the nucleotide sequence shown in SEQ ID NO: 5, and the antisense strand comprises the nucleotide sequence shown in SEQ ID NO: 6:

5'-CUGAAUUCCAAAAACCAA$Z_3$-3' (SEQ ID NO: 5);

5'-$Z_4$UUGGUUUUUGGAAUUCAGUA-3' (SEQ ID NO: 6),
or, the sense strand of the siRNA comprises the nucleotide sequence shown in SEQ ID NO: 7, and the antisense strand of the siRNA comprises the nucleotide sequence shown in SEQ ID NO: 8;
5'-UACUGAAUUCCAAAAACCAA$Z_3$-3' (SEQ ID NO: 7);
5'-$Z_4$UUGGUUUUUGGAAUUCAGUAUA-3' (SEQ ID NO: 8),
wherein, $Z_4$ is the first nucleotide from 5' terminal of the antisense strand; $Z_3$ is selected from A, U, G or C; and $Z_4$ is a nucleotide complementary to $Z_3$;
preferably, the siRNA is siPKKa1 or siPKKa2:

siPKKa1
sense strand: 5'-CUGAAUUCCAAAAACCAAU-3' (SEQ ID NO: 9);
antisense strand: 5'-AUUGGUUUUUGGAAUUCAGUA-3' (SEQ ID NO: 10),
siPKKa2
sense strand: 5'-UACUGAAUUCCAAAAACCAAU-3' (SEQ ID NO: 11);
antisense strand: 5'-AUUGGUUUUUGGAAUUCAGUAUA-3' (SEQ ID NO: 12).

**9.** The siRNA according to any one of claims 1-8, wherein at least one nucleotide in the sense strand or the antisense strand is a modified nucleotide, and/or at least one phosphate ester group is a phosphate ester group with modified group.

**10.** The siRNA according to any one of claims 1-9, wherein each nucleotide in the sense strand and the antisense strand is independently a fluoro modified nucleotide or a non-fluoro modified nucleotide.

**11.** The siRNA according to claim 10, wherein the fluoro modified nucleotides are located in the nucleotide sequence I and the nucleotide sequence II; and in the direction from 5' terminal to 3' terminal, at least the nucleotides at positions 7, 8, 9 of the nucleotide sequence I are fluoro modified nucleotides; and in the direction from 5' terminal to 3' terminal, at least the nucleotides at positions 2, 6, 14, 16 of the nucleotide sequence II are fluoro modified nucleotides;
preferably, in the direction from 5' terminal to 3' terminal, the nucleotides at positions 7, 8, 9 or at positions 5, 7, 8, 9 of the nucleotide sequence I in the sense strand are fluoro modified nucleotides, and the nucleotides at the rest of positions in the sense strand are non-fluoro modified nucleotides; and in the direction from 5' terminal to 3' terminal, the nucleotides at positions 2, 6, 14, 16 or at positions 2, 6, 8, 9, 14, 16 of the nucleotide sequence II in the antisense strand are fluoro modified nucleotides, and the nucleotides at the rest of positions in the antisense strand are non-fluoro modified nucleotides.

**12.** The siRNA according to claim 10 or 11, wherein each non-fluoro modified nucleotide is independently selected from one of a nucleotide formed by substituting the hydroxy at the 2'-position of the ribose group of a nucleotide with a non-fluoro group, or a nucleotide analogue.

**13.** The siRNA according to claim 12, wherein each non-fluoro modified nucleotide is a methoxy modified nucleotide, and the methoxy modified nucleotide refers to a nucleotide formed by substituting the 2'-hydroxy of the ribose group of a nucleotide with a methoxy.

**14.** The siRNA according to claim 9, wherein the phosphate ester group with modified group is a phosphorothioate group formed by substituting at least one oxygen atom in a phosphodiester bond of the phosphate ester group with a sulfur atom, and the linkage of phosphorothioate group exists in at least one of group consisting of the following positions:

the position between the first nucleotide and the second nucleotide at 5' terminal of the sense strand;
the position between the second nucleotide and the third nucleotide at 5' terminal of the sense strand;
the position between the first nucleotide and the second nucleotide at 3' terminal of the sense strand;
the position between the second nucleotide and the third nucleotide at 3' terminal of the sense strand;
the position between the first nucleotide and the second nucleotide at 5' terminal of the antisense strand;
the position between the second nucleotide and the third nucleotide at 5' terminal of the antisense strand;
the position between the first nucleotide and the second nucleotide at 3' terminal of the antisense strand; and
the position between the second nucleotide and the third nucleotide at 3' terminal of the antisense strand.

**15.** The siRNA according to any one of claims 1-14, wherein the nucleotide at the 5' terminal of the antisense strand is a 5'-phosphate nucleotide or a 5'-phosphate analogue modified nucleotide.

16. The siRNA according to any one of claims 1-15, wherein the siRNA is any one of siPKKa1-M1, siPKKa1-M2, siPKKa1-M3, siPKKa2-M1, siPKKa2-M2, siPKKa2-M3, siPKKa1-M1S, siPKKa1-M2S, siPKKa1-M3S, siPKKa2-M1S, siPKKa2-M2S, siPKKa2-M3S, siPKKa1-M1P1, siPKKa1-M2P1, siPKKa1-M3P1, siPKKa2-M1P1, siPKKa2-M2P1, siPKKa2-M3P1, siPKKa1-M1SP1, siPKKa1-M2SP1, siPKKa1-M3SP1, siPKKa2-M1SP1, siPKKa2-M2SP1 and siPKKa2-M3 SP1.

17. A pharmaceutical composition, wherein the pharmaceutical composition comprises the siRNA according to any one of claims 1-16 and a pharmaceutically acceptable carrier.

18. The pharmaceutical composition according to claim 17, wherein a weight ratio of the siRNA to the pharmaceutically acceptable carrier is 1:(1-500); and
preferably, the weight ratio of the siRNA to the pharmaceutically acceptable carrier is 1:(1-50).

19. An siRNA conjugate, wherein the siRNA conjugate comprises the siRNA according to any one of claims 1-16 and a conjugating group conjugated to the siRNA.

20. The siRNA conjugate according to claim 19, wherein the conjugating group comprises a pharmaceutically acceptable targeting group and a linker, and the siRNA, the linker and the targeting group are covalently or non-covalently linked in sequence.

21. The siRNA conjugate according to claim 19 or 20, wherein the siRNA conjugate has a structure as shown by Formula (403), (404), (405), (406), (407), (408), (409), (410), (411), (412), (413), (414), (415), (416), (417), (418), (419), (420), (421) or (422).

22. Use of the siRNA according to any one of claims 1-16, the pharmaceutical composition according to any one of claims 17-18, and/or the siRNA conjugate according to any one of claims 19-21 in the manufacture of a medicament for treating and/or preventing a pathological condition or disease caused by gene expression of plasma prekallikrein.

23. The use according to claim 22, wherein the pathological condition or disease caused by the gene expression of the plasma prekallikrein is selected from an inflammatory disease or an embolic disease or a physiological condition, especially hereditary angioedema.

24. A method for treating and/or preventing a pathological condition or disease caused by gene expression of plasma prekallikrein, wherein the method comprises administering an effective amount of the siRNA according to any one of claims 1-16, the pharmaceutical composition according to any one of claims 17-18, and/or the siRNA conjugate according to any one of claims 19-21 to a subject in need.

25. A method for inhibiting gene expression of plasma prekallikrein, comprising contacting an effective amount of the siRNA according to any one of claims 1-16, the pharmaceutical composition according to any one of claims 17-18, and/or the siRNA conjugate according to any one of claims 19-21 to the cell.

26. A kit, wherein the kit comprises the siRNA according to any one of claims 1-16, the pharmaceutical composition according to any one of claims 17-18, and/or the siRNA conjugate according to any one of claims 19-21.

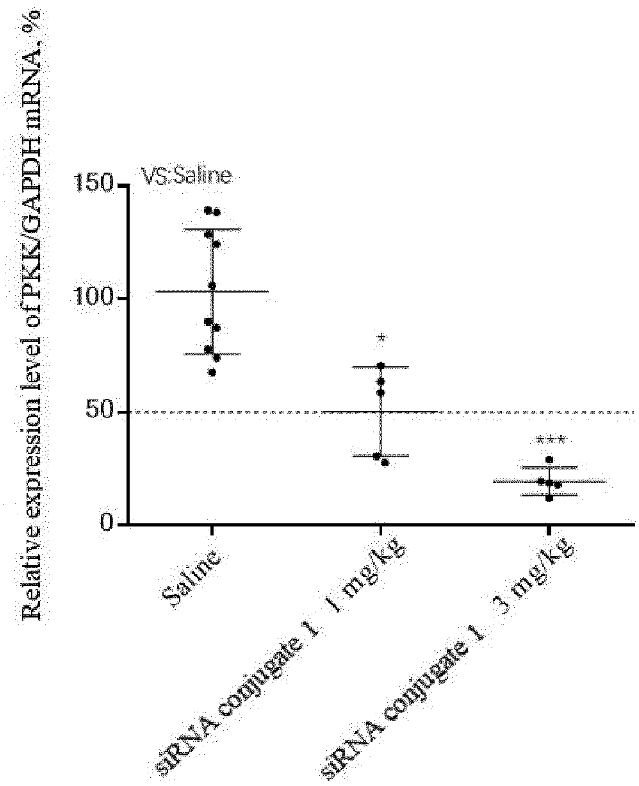
Relative expression level of PKK/GAPDH mRNA, %
VS:Saline
150
100
50
0
*
***
Saline
siRNA conjugate 1 1 mg/kg
siRNA conjugate 1 3 mg/kg

FIG. 1

## INTERNATIONAL SEARCH REPORT

International application No.
**PCT/CN2021/141665**

**A. CLASSIFICATION OF SUBJECT MATTER**

C12N 15/113(2010.01)i; A61K 31/713(2006.01)i; A61K 47/28(2006.01)i; A61P 9/10(2006.01)i

According to International Patent Classification (IPC) or to both national classification and IPC

**B. FIELDS SEARCHED**

Minimum documentation searched (classification system followed by classification symbols)

C12N A61K A61P

Documentation searched other than minimum documentation to the extent that such documents are included in the fields searched

Electronic data base consulted during the international search (name of data base and, where practicable, search terms used)

CNABS; CNTXT; CJFD; WPABS; WPABSC; ENTXTC; ENTXT; DWPI; CNKI; 万方, WANFANG; ISI Web of Science; STN; NCBI; 中国专利生物序列检索系统, China Patents Biological Sequence Search System: 苏州瑞博, 梁子才, 小干扰RNA, 前激肽释放酶, 抑制, SUZHOU RIBO, Liang zicai, small interfering RNA, siRNA, PKK, plasma prekallikrein, Inhibit, 序列1-80

**C. DOCUMENTS CONSIDERED TO BE RELEVANT**

| Category* | Citation of document, with indication, where appropriate, of the relevant passages | Relevant to claim No. |
|---|---|---|
| A | WO 2020238758 A1 (SUZHOU RIBO LIFE SCIENCE CO., LTD.) 03 December 2020 (2020-12-03)<br>description, page 104, comparative embodiment 1, description, pages 108 and 109, embodiment 3, and description, page 98, table 4, and figures 1-3 | 1-23, 25, 26 |
| A | CN 106232125 A (IONIS PHARMACEUTICALS, INC.) 14 December 2016 (2016-12-14)<br>entire document | 1-23, 25, 26 |
| A | CN 105517556 A (IONIS PHARMACEUTICALS, INC.) 20 April 2016 (2016-04-20)<br>entire document | 1-23, 25, 26 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 March 2022** | **14 March 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/141665**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2021/141665**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **24、25**
because they relate to subject matter not required to be searched by this Authority, namely:

[1] Claim 24 relates to a method for treating and/or preventing a pathological condition or disease caused by genetic expression of plasma prekallikrein. Claim 25 relates to a method for inhibiting genetic expression of plasma prekallikrein. The technical solutions are diagnostic methods implemented on a living human or animal body, and fall within the subject matter as defined in PCT Rule 39.1(iv) that does not warrant a search conducted by the international searching authority.

[2] The following amendment is reasonably expected for claim 25:

[3] a method for inhibiting genetic expression of plasma prekallikrein, comprising contacting an effective amount of siRNA according to any one of claims 1-16, pharmaceutical composition according to any one of claims 17 and 18, and/or siRNA conjugate according to any one of claims 19-21 with ex vivo cells.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2021/141665**

| Patent document cited in search report | Publication date (day/month/year) | Patent family member(s) | Publication date (day/month/year) |
|---|---|---|---|
| WO 2020238758 A1 | 03 December 2020 | None | |
| CN 106232125 A | 14 December 2016 | ES 2849600 T3 | 19 August 2021 |
| | | AU 2019284028 A1 | 23 January 2020 |
| | | JP 2017518045 A | 06 July 2017 |
| | | JP 6665111 B2 | 13 March 2020 |
| | | US 2020056185 A1 | 20 February 2020 |
| | | HU E052709 T2 | 28 May 2021 |
| | | JP 2020096629 A | 25 June 2020 |
| | | DK 3137091 T3 | 25 January 2021 |
| | | WO 2015168532 A2 | 05 November 2015 |
| | | WO 2015168532 A3 | 14 April 2016 |
| | | KR 20160147891 A | 23 December 2016 |
| | | BR 112016022855 A2 | 17 October 2017 |
| | | IL 273058 D0 | 30 April 2020 |
| | | MX 2016014140 A | 15 September 2017 |
| | | EP 3862362 A2 | 11 August 2021 |
| | | EP 3862362 A3 | 27 October 2021 |
| | | US 2017183661 A1 | 29 June 2017 |
| | | US 10294477 B2 | 21 May 2019 |
| | | RU 2016146818 A | 04 June 2018 |
| | | RU 2016146818 A3 | 26 October 2018 |
| | | RU 2703411 C2 | 16 October 2019 |
| | | CA 2943705 A1 | 05 November 2015 |
| | | IL 247862 D0 | 30 November 2016 |
| | | IL 247862 A | 31 March 2020 |
| | | AU 2015252917 A1 | 29 September 2016 |
| | | AU 2015252917 B2 | 26 September 2019 |
| | | EP 3137091 A2 | 08 March 2017 |
| | | EP 3137091 A4 | 27 December 2017 |
| | | EP 3137091 B1 | 02 December 2020 |
| | | US 2021277401 A1 | 09 September 2021 |
| | | IN 201647041036 A | 05 May 2017 |
| | | CN 106232125 B | 16 October 2020 |
| | | MX 377141 B | 05 November 2020 |
| | | HK 1234330 A0 | 15 February 2018 |
| CN 105517556 A | 20 April 2016 | US 2019233827 A1 | 01 August 2019 |
| | | US 11053500 B2 | 06 July 2021 |
| | | JP 2016533751 A | 04 November 2016 |
| | | JP 6652922 B2 | 26 February 2020 |
| | | BR 112016004093 A2 | 17 October 2017 |
| | | AU 2014312196 A1 | 03 March 2016 |
| | | AU 2014312196 B2 | 18 April 2019 |
| | | RU 2016110848 A | 27 November 2018 |
| | | RU 2016110848 A3 | 27 November 2018 |
| | | RU 2712559 C2 | 29 January 2020 |
| | | RU 2712559 C9 | 08 October 2020 |
| | | EP 3715457 A2 | 30 September 2020 |
| | | EP 3715457 A3 | 16 December 2020 |
| | | WO 2015031679 A2 | 05 March 2015 |
| | | WO 2015031679 A3 | 14 May 2015 |

Form PCT/ISA/210 (patent family annex) (January 2015)

**INTERNATIONAL SEARCH REPORT**
**Information on patent family members**

International application No.
**PCT/CN2021/141665**

| Patent document cited in search report | Publication date (day/month/year) | Patent family member(s) | | | Publication date (day/month/year) |
|---|---|---|---|---|---|
| | | AU | 2019204977 | A1 | 01 August 2019 |
| | | US | 2017002359 | A1 | 05 January 2017 |
| | | US | 9670492 | B2 | 06 June 2017 |
| | | JP | 2020072732 | A | 14 May 2020 |
| | | HK | 1225639 | A1 | 15 September 2017 |
| | | CA | 2921839 | A1 | 05 March 2015 |
| | | MX | 2016002587 | A | 20 February 2017 |
| | | US | 2017314026 | A1 | 02 November 2017 |
| | | US | 10100310 | B2 | 16 October 2018 |
| | | ES | 2790574 | T3 | 28 October 2020 |
| | | IL | 244047 | D0 | 21 April 2016 |
| | | EP | 3038627 | A2 | 06 July 2016 |
| | | EP | 3038627 | A4 | 17 May 2017 |
| | | EP | 3038627 | B1 | 04 March 2020 |
| | | KR | 20160048119 | A | 03 May 2016 |
| | | IL | 244047 | A1 | 31 March 2016 |
| | | IN | 201647009692 | A | 31 August 2016 |
| | | AU | 2014312196 | C1 | 14 November 2019 |
| | | CN | 105517556 | B | 02 February 2021 |
| | | HK | 1225639 | A0 | 15 September 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103380113 A
- WO 2009082607 A2
- CN 105378082 A
- WO 2015006740 A2
- WO 2014025805 A1
- CN 110959011 A


### Non-patent literature cited in the description


- **BEAUCAGE et al.** *Tetrahedron,* 1992, vol. 48, 2223-2311
- **GREENE ; WUTS.** Protective Groups in Organic Synthesis. John Wiley & Sons, 1991
- **J.K. WATTS ; G. F. DELEAVEY ; M. J.DAMHA.** *Chemically Modified siRNA: tools and applications. Drug Discov Today,* 2008, vol. 13 (19-20), 842-855
- *Nature Biotechnology,* 2017, vol. 35 (3), 238-248
- *ACS Chemical biology,* 2015, vol. 10 (5), 1181-1187
- **RAJEEV et al.** *ChemBioChem,* 2015, vol. 16, 903-908
- Molecular Cloning. Cold Spring Harbor Laboratory Press, 1989